# EUROPEAN PATENT APPLICATION

(11) **EP 4 806 419 A2**
(43) Date of publication of application: **16.09.2026**
(21) Application number: 26195270.9
(22) Date of filing: 04.06.2021
(51) Int. Cl.: A61K 8/49

(54) **CONSUMER PRODUCTS WITH IMPROVED AESTHETICS**

(30) Priority: 05.06.2020 US 202063035176 P
(62) Document number(s) of the earlier application(s) in accordance with Art. 76 EPC: 21177705.7 / 3 970 690
(71) Applicant: International Flavors & Fragrances, Inc., New York, NY 10019 (US)
(72) Inventor: TEIXEIRA, Miguel A., Porto (PT); SCHIET, Franc T., Hilversum (NL); KERSCHNER, Judith Lynne, Holmdel, New Jersey (US); DORO, Franco, Hilversum (NL); YOUNG, Timothy, Union Beach, New Jersey (US); DE JESUS, Jill Kristine Calindas, Holmdel, New Jersey (US); FAN, Yan Liang Betty, Singapore (SG); HUNTER, Robert Allan, Hilversum (NL); DE VILLENEUVE, Voklert, Hilversum (NL); GIROD, Caroline Celine Rose, Hilversum (NL); BRAIN, Joseph, Holmdel, New Jersey (US); GIFFIN, Nicole, Hazlet, New Jersey (US); GENO, Jason, Holmdel, New Jersey (US); BRAHMS, John, Morris Plains, New Jersey (US)
(74) Representative: International N&H EMEA

(57) **Abstract**

A consumer product composed of ≤1% of a High-Performance fragrance composition and a consumer product active is provided. Use of reduced levels of the High-Performance fragrance composition provides for improved aesthetics such as improved clarity and viscosity, and reduced discoloration and caking.

## Description

### Background

Fragrance compositions are applied in consumer products to deliver experiential and functional benefits to consumers. However, the incorporation of fragrances into formulations can introduce both chemical and physical adversities in the final products. Specifically, for physical adversities, the addition of fragrance can result in poor product transparency in low surfactant formulations and product viscosity fluctuation in liquid applications. For low surfactant formulations, such as self-foaming base, the incorporation of fragrance may result in final product with turbid aspect. To overcome this issue, solubilizers are typically added to the fragrances to ensure better solubilization, and thus to obtain clear and/or transparent products.

### Summary of the Invention

This invention provides a consumer product with an improved aesthetic, wherein said consumer product is composed of ≤ 1% of a high-performance fragrance composition and a consumer product active. In one aspect, the high performance fragrance composition includes at least 55% (e.g., at least 60%, at least 75%, and at least 90%) by weight of one or more (*e.g.*, two or more, five or more, and seven or more) high-performance fragrance ingredients listed in Table 1 or Table 2 below. Consumer products of use in this invention include personal care products, fabric care products, or home fragrance products. In embodiments where the consumer product is a body wash, said consumer product exhibits a clarity of less than 20 Nephelometric Turbidity Units, a feature that is maintained for at least a month after storage at 45°C. In embodiments where the consumer product is a body wash, said consumer product exhibits a viscosity in the range of 10000 and 12000 mPas, a feature that is maintained for at least a month after storage at 45°C. In embodiments where the consumer product is a scent booster or liquid detergent, said consumer product exhibits reduced discoloration. In embodiments where the consumer product is a powder detergent, said consumer product exhibits reduced caking. In embodiments where the consumer product is a fabric conditioner, the consumer product active is present at a level between 1% and 20% by weight of the consumer product. In embodiments where the consumer product is a candle, said consumer product exhibits reduced soot and volatile organic compound production.

### Brief Description of the Drawings

FIG. 1A-1B show viscosity measurements of a shower gel containing regular and high-performance (HP) fragrances initially after preparation and after one-month storage at 45°C, respectively.
FIG. 2 shows the sensory evaluation results of three fragrances at dry pre and dry gentle handling at fresh. Connecting letters reports are provided above each bar. Levels that share, or are connected by, the same letter do not differ statistically. Levels that are not connected by a common letter do differ statistically.
FIG. 3 shows the performance results for a high-performance fragrance (High-performance (HP) 3) compared to a benchmark fragrance at different active levels. Connecting letters reports are provided above each bar.

### Detailed Description of the Invention

It has now been found that the development of fragrance compositions that adhere to a set of guidelines for the inclusion of particular types of fragrances, results in an ultra-high performing/impact fragrance composition that improves aesthetic properties of consumer products. In particular, the creation, or modification, of a fragrance composition to include at least 60% by weight of an high-performance fragrance can result in a fragrance composition that can deliver parity or superior performance at dosages that are five- to ten-times lower than the standard fragrance usage levels. In addition to the performance benefit at lower fragrance dosage, product clarity or transparency in low surfactant formulations (*e.g.,* self-foaming base) is improved; product viscosity fluctuations in personal wash liquid formulations (*e.g.,* shower gels) are reduced; the need for solubilizers is reduced or eliminated; melting point of solid scent booster compositions is increased thereby and improving physical stability; product discoloration due to fragrance is decreased; usage levels of scent booster products can be reduced while still achieving the same fragrance intensity; caking of powder detergents is reduced; and soot and volatile organic compound production by candles is decreased.

This invention therefore provides a fragrance composition for use at a level of less than or equal to 1% by weight in a consumer product thereby improving one or more aesthetic properties of the consumer product while maintaining the desired fragrance performance. For the purposes of the present invention, the terms "fragrance composition," "fragrance formulation," and "perfume composition" mean the same and refer to a composition that is a mixture of fragrance ingredients including, for example, alcohols, aldehydes, ketones, esters, ethers, lactones, nitriles, natural oils, synthetic oils, and mercaptans, which are admixed so that the combined odors of the individual ingredients produce a pleasant or desired fragrance.

In certain aspects, the fragrance composition is composed of one or a combination of fragrances, wherein at least one of said fragrances is a high-performance fragrance. More specifically, the fragrance composition includes at least about 60% (or 65%, 70%, 75%, 80%, 85%, 90%, 95% or 99%) by weight of a high-performance fragrance. As used herein, the term "about" is intended to refer to an amount ± 0.01% to 0.5% of the amount specified. Any one of the above-referenced fragrances may also be present within any range delimited by any pair of the foregoing values, such as between 5% and 50%, between 40% and 60% or between 60% and 90%, for example.

In some embodiments, a fragrance composition includes at least about 60% (*e.g.*, at least 75%, at least 80%, or at least 90%) by weight of one or more High-Performance fragrance ingredients.

For the purposes of this invention, High-Performance fragrance ingredients of use either alone or in combination in the fragrance composition are selected from the fragrance ingredients listed in Table 1.

**TABLE 1**

| |
|---|
| (1R,2R,4R)-ethyl bicyclo[2.2.1]hept-5-ene-2-carboxylate |
| (2E,6Z)-nonadienal |
| (2E,6Z)-nonadienol |
| (2-methoxyethyl)benzene |
| (2R,4S)-2-methyl-4-propyl-1,3-oxathiane |
| (2R,4S)-4-methyl-2-(2-methylprop-1-en-1-yl)tetrahydro-2H-pyran |
| 3,7-dimethyl-2,6-octadienal |
| (3aR-(3aalpha,5abeta,9aalpha,9bbeta))-dodecahydro-3a,6,6,9a-tetramethyl naphtha(2,1-b)furan |
| (3aR,8bS)-2,2,6,6,7,8,8-heptamethyldecahydro-2H-indeno[4,5-b]furan |
| (4aR,5R,7aS,9R)-octahydro-2,2,5,8,8,9a-hexamethyl-4h-4a,9-methanoazuleno(5,6-d)-1,3-dioxole (ambrocenide, CAS No. 211299-54-6) |
| (9S,1R)-5,5,9,13-tetramethyl-14,16-dioxatetracyclohexadecane |
| (all-E)-alpha-sinensal |
| (Z)-geranyl nitrile (CITRALVA^{®} Plus, CAS No. 31983-27-4) |
| 1-(2,6,6-trimethyl-1,3-cyclohexandienyl)-2-buten-1-one |
| 1-(2,6,6-trimethyl-3-cyclohexen-1-yl)-2-Buten-1-one) |
| 1-(3-methylbenzofuran-2-yl)ethan-1-one |
| 1-(5,5-dimethyl-1-cyclohexen-1-yl)-4-Penten-1-one |
| 1-(ethoxymethyl)-2-methoxybenzene (rosethyl, CAS No. 64988-06-3) |
| 1,1-dimethoxy-2,2,5-trimethyl-4-hexene |
| 1,1'-oxybis-benzene |
| 1,2,3,4,5,6,7,8-octahydro-1,1,6,7-tetramethyl naphthalene |
| 1,2,3,4~{a},5,6-hexahydronaphthalene, 4,4,7,8~{a}-tetramethyl-8-(3-methylpent-4-enyl)- |
| 1,3,3-trimethyl-2-oxabicyclo[2.2.2]octane |
| 1,3,4,6,7,8-hexahydro-4,6,6,7,8,8-hexamethylcyclopenta-gamma-2-benzopyrane |
| 1,3-oxathiane |
| 1,3-oxathiane, (2~{R},4~{S})-2-methyl-4-propyl- |
| 1,6,10-dodecatriene, 7,11-dimethyl-3-methylene-, (E)- |
| 1,8-cineole (CAS No. 470-82-6) |
| 1~{a},2,3,4,4~{a},5,6,7~{b}-octahydrocyclopropa[e]azulene, (1~{a}~{R},4~{R},4~{a}~{R},7~{b}~{S})-1,1,4,7-tetramethyl- |
| 10-undecen-1-al |
| 1-cyclohexanecarboxylic acid, 3-methyl-, methyl ester |
| 1-cyclohexanone, (2~{S},5~{S})-2-(2-mercaptopropan-2-yl)-5-methyl- |
| 1-hexadecene, 7,11,15-trimethyl-3-methylene- |
| 1-methoxy-4-methylbenzene |
| 1-methyl-4-(prop-1-en-2-yl)cyclohex-1-ene |
| 1-octen-3-ol |
| 1-terpinen-4-ol |
| 2-(2-(4-methyl-3-cyclohexan-1-yl)-cyclopentanone |
| 2-(2-(4-methyl-3-cyclohexen-1-yl)propyl)-cyclopentanone |
| 2-(3-phenylpropyl)pyridine |
| 2-(cyclododecyl)-propan-1-ol |
| 2,2'-(dithiodimethylene)di-furan |
| 2,2,5-trimethyl-5-pentylcyclopentan-1-one (VELOUTONE^{®}) |
| 2,3,5-trimethylpyrazine |
| 2,3-dimethylpyrazine |
| 2,3-dimethyphenol |
| 2,4,6-trimethyl 3-cyclohexene-1-carboxaldehyde |
| 2,4-decadienoic acid, ethyl ester (E,Z)- |
| 2,4-dimethyl-2-(1,1,4,4-tetramethyl)tetralin-6-yl)-1,3-dioxolane |
| 2,4-dimethyl-3-cyclohexene-1-carbaldehyde |
| 2,4-dimethylphenol |
| 2,5-dimethylpyrazine |
| 2,6,10-trimethyl-2(E),6(E),9(E)-11-dodecatetraenal |
| 2,6,10-trimethyl-9-undecenal |
| 2,6,6-trimethylbicyclo[3.1.1]hept-2-ene |
| 2,6-dimethyl-2,6-octadien-8-ol |
| 2,6-dimethyl-5-heptenal |
| 2,6-dimethylhept-5-enal (Melonal, CAS No. 106-72-9) |
| 2,6-dimethylheptan-2-ol |
| 2,6-dimethylpyrazine |
| 2,6-nonadien-1-ol |
| 2,6-nonadienal |
| 2,6-nonadienal diethyl acetal |
| 2,6-nonadienenitrile |
| 2,6-nonenol |
| 2,6-octadienenitrile, 3,7-dimethyl-, (Z)- |
| 2-acetylyyridine 10% IPM |
| 2-bicyclo[2.2.1]hept-5-enecarboxylic acid, (2~{R})-, ethyl ester |
| 2-buten-1-one, 1-(2,6,6-trimethyl-1,3-cyclohexadien-1-yl)- |
| 2-buten-1-one, 1-(2,6,6-trimethyl-2-cyclohexen-1-yl)-, (E)- |
| 2-decenal |
| 2-ethyl-3,5-dimethylpyrazine |
| 2-ethyl-3-methylpyrazine |
| 2-hepten-4-one, (~{E})-5-methyl- |
| 2-heptonone |
| 2H-pyran, 3,6-dihydro-4-methyl-2-(2-methyl-1-propenyl)- |
| 2H-pyran, tetrahydro-4-methyl-2-(2-methyl-1-propenyl)- |
| 2H-pyran-2-one, tetrahydro-6-(3-pentenyl)- |
| 2-isobutyl-3-methoxypyrazine |
| 2-isobutylthiazole |
| 2-isopropyl-4-methylthiazole |
| 2-methoxy-3-(1-methylpropyl) pyrazine |
| 2-methoxy-4-(2-propenyl)phenol |
| 2-methoxy-4-methyl phenol |
| 2-methoxy-4-vinyl phenol (varamol-106, CAS No. 7786-61-0) |
| 2-methoxy-4-vinylphenol |
| 2-methyl butyric acid (CAS No. 116-53-0) |
| 2-methyl-3-(para-iso-propylphenyl)propionaldehyde |
| 2-methylbutyric acid |
| 2-methylundecanal |
| 2-nonen-1-al |
| 2-nonenenitrile |
| 2-nonenenitrile, (~{E})- |
| 2-oxiranecarboxylic acid, 3-phenyl-, ethyl ester |
| 2-oxolanone, 5-hexyl- |
| 2-pentenoic acid, (~{E})-2-methyl- |
| 2-pentyl furan |
| 2-pentylcyclopentanone |
| 2-pentylfuran |
| 2-phenylethyl acetate |
| 2-phenylethylalcohol |
| 2-propanethiol, 2-(4-methyl-1-cyclohex-3-enyl)- |
| 2-propenoic acid, (~{E})-3-phenyl-, [(~{E})-3-phenylprop-2-enyl] ester |
| 2-propenyl ester |
| 2-propenyl para cymene |
| 2-trans 6-cis nonadienol |
| 2-tricyclo[5.2.1.0^{2,6}]decanecarboxylic acid, ethyl ester |
| 2-tridecenal, (~{E})- |
| 3-(3-isopropylphenyl)butanal |
| 3,3,5-trimethylcyclohexanol |
| 3,3-dimethyl-5-(2,2,3-trimethyl-3-cyclopenten-1-yl)-4-pentene-2-ol |
| 3,6-dihydro-2~{H}-pyran, 4,6-dimethyl-2-(1-phenylethyl)- |
| 3,6-dihydro-4,6-dimethyl-2-(1-phenylethyl)-2H-pyran |
| 3,6-nonadienol |
| 3,7-dimethyl-1,6-octadien-3-ol |
| 3,7-dimethyl-2,6-octadienal |
| 3,7-dimethyl-2,6-octadienenitrile |
| 3,7-dimethyl-6-octen-1-ol |
| 3,7-dimethyl-octanal |
| 3a,4,5,6,7,7a-hexahydro-3H-benzofuran-2-one, 3-ethyl- |
| 3-buten-2-one |
| 3-cyclohexene-1-carboxaldehyde |
| 3-decen-5-one, (~{E})-4-methyl- |
| 3-dodecenal |
| 3-ethyl-2,5-dimethylpyrazine |
| 3-furanone, 4-hydroxy-2,5-dimethyl- |
| 3-furanone, 5-ethyl-4-hydroxy-2-methyl- |
| 3-hydroxy-2-methyl-4-pyrone |
| 3-methyl-(cis-2-penten-1-yl)-2-cyclopenten-1-one |
| 3-methyl-2-buten-1-yl acetate |
| 3-methyl-4(5)-cyclopentadecenone |
| 3-methyl-5-(2,2,3-trimethyl-3-cyclopenten-1-yl)pent-4-en-2-ol (EBANOL^{®}) |
| 3-methyl-5-propyl-cyclohexen-1-one |
| 3-p-cumenyl-propionaldehyde |
| 3-tricyclo[5.3.1.03,8]undecanol, 2,2,6,8-tetramethyl- |
| 4-(1-methylethyl)-benzenepropanal |
| 4-(2,2,6-Trimethylcyclohex-1-enyl)-2-but-en-4-one |
| 4-(2,6,6-Trimethyl-1,3-cyclohexadienyl)-3-buten-4-one |
| 4-(2,6,6-trimethyl-1-cyclohexen-1-yl)-3-buten-2-one |
| 4-(4-methylpent-3-enyl)cyclohex-3-ene-1-carbaldehyde (myrac aldehyde, CAS No. 37677-14-8) |
| 4-(heptyloxy)-3-methylbutanal (CRISTALFIZZ) |
| 4,5,7,8b-tetrahydro-3aH-cyclopenta[e][1,3]benzodioxole, 2,2,6,6,7,8,8-heptamethyl- |
| 4,6-dimethylcyclohex-3-enecarbaldehyde |
| 4,7-methanoindan 1-carboxaldehyde, hexahydro |
| 4-acetyl-6-tertiarybutyl-1,1-dimethyl indan |
| 4-dodecenal |
| 4-methoxybenzaldehyde (anisaldehyde, anisic aldehyde, CAS No. 123-11-5) |
| 4-methyl-3-decen-5-ol |
| 4-methyl-4-mercaptopentan-2-one |
| 4-methylene-2-phenyltetrahydro-2H-pyran |
| 4-nonanolide |
| 4-penten-1-one |
| 4-pentene-2-ol |
| 4-pyranone, 2-ethyl-3-hydroxy- |
| 5,6,7,8-tetrahydroquinoxaline |
| 5,6,7,8-tetrahydroquinoxaline |
| 5,7-dihydrothieno[3,4-d]pyrimidine, 2-methyl- |
| 5-cyclohexadecenone-1 |
| 5-methyl-2-(1-methylethyl)cyclohexanone |
| 5-methyl-3-heptanone oxime |
| 6-(and 8-) isopropylquinoline |
| 6-(Z,3-pentenyl)-tetrahydro-(2H)-pyranone-2 |
| 6,6-dimethyl-2-norpinene-2-propionaldehyde |
| 6,6-dimethylbicyclo(3.1.1)Hept-2-ene-2-proponal |
| 6,7-dihydro-1,1,2,3,3-Pentamethyl-4(5H)-indanone |
| 6,8-dimethyl-2-nonanol |
| 6-acetyl-1,1,3,4,4,6-hexamethyl tetrahydro naphthalene |
| 6-decenal, (~{E})- |
| 6-methylquinoline |
| 6-nonen-1-ol |
| 7,7,8,9,9-pentamethyl-6,6a,7,8,9,9a-hexahydro-5H-cyclopenta[h]quinazoline (Ambertonic) |
| 7,7,8,9,9-pentamethyl-6,7,8,9-tetrahydro-5H-cyclopenta[h]quinazoline (Sinfonide) |
| 7-acetyl-1,2,3,4,5,6,7,8-octahydro-1,1,6,7-tetramethyl naphthalene |
| 8alpha,12-oxido-13,14,15,16-tetranorlabdane |
| 8-cyclohexadecen-1-one |
| 8-spiro[4.5]dec-9-enone, 6,10-dimethyl-3-propan-2-ylidene- |
| 9-decen-1-ol |
| 9-decen-1-ol (rosalva, CAS No. 13019-22-2) |
| 9-decenal |
| Abhexone, 5-ethyl-3-hydroxy-4-methyl-2(5H)-furanone (CAS No. 698-10-2) |
| acetaldehyde phenylethyl propyl acetal |
| acetaldehyde, [(3,7-dimethyl-6-octenyl)oxy]- |
| acetic acid, (2-methylbutoxy)-, 2-propenyl ester |
| acetic acid, (3-methylbutoxy)-, 2-propenyl ester |
| acetic acid, [(1~{R},5~{S})-7,7-dimethyl-6-methylene-2-tricyclo[6.2.1.0^{1,5}]undecanyl]methyl ester |
| acetic acid, [5-(2,5,5,8~{a}-tetramethyl-3,4,4~{a},6,7,8-hexahydronaphthalen-1-yl)-3-methylpentyl] ester |
| acetic acid, 2-phenyl- |
| acetic acid, 2-phenylethyl ester |
| acetic acid, 4-methylphenyl ester |
| acetophenone |
| acetyl methyl carbinol (acetoin) |
| adoxal |
| a-irone |
| aldehyde C-11 INTRELEVEN (TT) PRG |
| aldehyde C-11 MOA BHT |
| aldehyde C-11 ULENIC TOCO |
| aldehyde C-11 UNDECYLIC TOCO |
| aldehyde C-12 LAURIC TOCO |
| aldehyde C-12 MNA TOCO |
| aldehyde C-16 STRAWB#2 |
| aldehyde C-18 |
| aldehyde C-6 Toco |
| aldehyde C-7 Stabiliff |
| aldehyde C-8 TOCO |
| aldehyde C-9 TOCO |
| aldehyde supra (mandarine undecenal, CAS No. 143-14-6) |
| allyl amyl glycolate |
| allyl caprylate |
| allyl cyclohexyl propionate |
| allyl heptanoate |
| allyl heptoate |
| allyl hexanoate (allyl caproate, CAS No. 123-68-2) |
| alpha beta ionone |
| alpha damascone damascene |
| alpha ionone |
| alpha-ambrinol (1,2,3,4,4a,5,6,7-octahydro-2,2,5-trimethyl-2-naphthalenol, CAS No. 41199-19-3) |
| alpha-damascone |
| alpha-terpineol |
| Amber Xtreme (decahydro-2,2,6,6,7,8,8-heptamethyl indenofuran, CAS No. 476332-65-7) |
| ambergris |
| Amberketal 8.5% IPM (amber oxepin, CAS No. 57345-19-4) |
| ambrettolide (omega-6-hexadecenlactone, 17-oxacycloheptadec-6-en-1-one, CAS No. 7779-50-2) |
| ambroxan (AMBERMOR^{®} EX, abbrox, (3aR,5aS,9aS,9bR)-3a,6,6,9a-Tetramethyldodecahydronaphtho[2,1-b]furan, CAS No. 6790-58-5) |
| amyl acetate |
| amyl cinnamic aldehyde |
| amyl salicylate |
| Amyris oil |
| anethol (CAS No. 104-46-1) |
| Angelica root oil |
| Angelica seed oil |
| anisaldehyde diethyl acetal |
| Anise seed oil Spanish |
| anisic alcohol (CAS No. 105-13-5) |
| AQUAFLORA^{®} toco (octahydro-4,7-methano-1H-indene-5-acetaldehyde, CAS Nos. 1339119-15-1 and 1338815-87-4) |
| armoise ess robt (CAS No. 68991-20-8) |
| armoise oil pure |
| asafetida root oil |
| aurantiol (methyl 2-[(7-hydroxy-3,7-dimethyloctylidene)amino]benzoate, CAS 89-43-0) |
| bacdanol |
| basil oil (absolute, grand, sweet) |
| benzaldehyde |
| benzene, 1-methoxy-4-methyl- |
| benzene, 2-methoxyethyl- |
| benzene, phenoxy- |
| benzenepropanal, 4-(1,1-dimethylethyl)- |
| benzoic acid, (phenylmethyl) ester |
| benzoic acid, (phenylmethyl) ester |
| benzoic acid, 2,4-dihydroxy-3-methyl-, methyl ester |
| benzoic acid, 2-hydroxy-, ethyl ester |
| benzoic acid, 2-hydroxy-, methyl ester |
| benzyl acetate |
| benzyl alcohol and ester derivatives |
| benzyl benzoate |
| benzyl iso eugenol |
| benzyl propionate |
| benzyl salicylate |
| bergamot oil |
| beta gamma hexanol |
| beta naphthyl ethyl ether |
| beta-Ionone |
| beta-Pinene |
| Bigarade oxide |
| Birch Tar Oil (Rectified, CAS No. 8001-88-5) |
| black pepper oil |
| Blackcurrant Bud Absolute |
| borneol |
| bourgeonal |
| buccoxime |
| Buchu leaf oil (Betulina Natural, CAS No. 68650-46-4) |
| buranoic acid, 2-methyl-, 3-hexenyl ester, (Z)- |
| butanal, 4-(8-tricyclo[5.2.1.02,6]decanylidene)- |
| butanoic acid, 2-methyl-, ethyl ester |
| butanoic acid, 2-methyl-, ethyl ester |
| butyl acetate |
| cade oil (rectified, CAS No. 8013-10-3) |
| Calone |
| camphor |
| carbitol |
| cardamom oil (pure, extract, absolute) |
| carrot seed extract |
| carvone |
| CASHMERAN^{®} ((RS)-1,1,2,3,3-Pentamethyl-1,2,3,5,6,7-hexahydro-4H-inden-4-one) |
| cassis ether |
| castoreum (absolute, resoid, oil) |
| Cedarwood oil |
| CEDRAMBER^{®} (Cedryl Methyl Ether) |
| CETALOX^{®} (CAS No. 3738-00-9) |
| chamomile oil (English, Roman, wild, extract, absolute, CAS No. 8015-92-7) |
| cinnamic alcohol |
| cinnamic aldehyde |
| cinnamon bark oil (extract, absolute, essential, CAS No. 8015-91-6) |
| cinnamon extract |
| cinnamyl acetate |
| cinnamyl alcohol |
| cis jasmone |
| cis-3-hexenol |
| cis-3-hexenyl acetate |
| cis-3-Hexenyl butyrate |
| cis-3-hexenyl methyl carbonate |
| cis-3-hexenyl propionate |
| cis-3-hexenyl salicylate |
| cis-4-decenal |
| cis-6-nonadienol |
| cis-6-nonen-1-al |
| cis-6-nonenol |
| citral |
| citrathal |
| citronella oil |
| citronellal |
| citronellol and ester derivatives |
| citronellyl nitrile (agruntitrile, CAS No. 51566-62-2) |
| citronellyloxyacetaldehyde |
| citrylal (lime octadienal, CAS No. 147060-73-9) |
| civet |
| clonal (dodecane nitrile, CAS No. 2437-25-4) |
| clove oil |
| Coffee Absolute Arabica oil (CO2) |
| Coffee oil FILT BRAZ. |
| Coolwood |
| copaiba balsam |
| corps cassis 0.1% TEC |
| Corps Pamplemousse Pure (CAS No. 68398-18-5) |
| Corps Racine Vs 600164 Conc |
| Costasid |
| coumarin |
| creosol |
| cumin aldehyde |
| cumin seed oil |
| CYCLACET^{®} (verdyl acetate; CAS No. 5413-60-5) |
| cyclamen aldehyde |
| cyclemax |
| cyclo galbanate |
| cyclohexa-1,3-diene, 2-methyl-5-propan-2-yl- |
| Cyclohexane Propanol 2,2,6-Trimethyl-Alpha,Propyl |
| cyclopropanecarboxylic acid, [(~{Z})-hex-3-enyl] ester |
| Cymal |
| Cypress oil |
| Cypriol oil |
| damarose Alpha |
| damascene |
| damascenone |
| damascones |
| Dartanol ((-)-(1'r,E)-2-Ethyl-4-(2',2',3'-Trimethyl-3'-Cyclopenten-1'-yl)-2-Buten-1-ol) |
| Davana oil |
| D-carvone (CAS No. 2244-26-8) |
| deca-4,9-dienal, (4E)-4,8-dimethyl- |
| decalactone gamma |
| decanal (ALD C-10) |
| decanol |
| delphone |
| delta damascone |
| delta muscenone |
| delta-muscenone (MUSCEMOR^{®}) |
| dextro limonene |
| diacetyl |
| dicyclopentadiene propionate (CYCLAPROP^{®}, CAS 68912-13-0) |
| dihydro iso jasmonate |
| dihydro-beta-ionone |
| dihydromyrcenol (CAS No. 18479-58-8) |
| dihydro-nor-dicyclopentadienyl acetate |
| dihydro-nor-dicyclopentadienyl propionate |
| dimethyl benzyl carbinol |
| dimethyl benzyl carbinyl butyrate |
| dimethyl sulfide |
| dimethylindane derivatives |
| dimetol |
| dipropylene glycol monomethyl ether (1-(2-eethoxypropoxy)-2-propanol) |
| d-limonene |
| dodecanal |
| Dowanol TPM |
| DYNASCONE^{®} Pure BHT (galbascone, CAS No. 56973-85-4) |
| ethyl acetoacetate |
| estragole |
| ethanol, 2-(4-methyl-5-thiazolyl)- |
| ethyl 2,4-decadienoate |
| Ethyl 2-methyl-1,3-dioxolane-2-acetate (Fructone) |
| ethyl 2-methylbutyrate |
| ethyl 2-methylpentanoate (applinate, CAS No. 39255-32-8) |
| ethyl acetate |
| ethyl anthranilate |
| ethyl butyrate |
| ethyl caproate |
| ethyl caprylate |
| ethyl cinnamate |
| ethyl heptanoate |
| ethyl hexanoate |
| ethyl isobutyrate |
| ethyl lactate |
| ethyl linalool |
| ethyl methyl dioxolane acetate |
| ethyl methyl phenyl glycidate |
| ethyl oenanthate |
| ethyl propionate |
| ethyl valerate |
| ethyl vanillin |
| ethyl-2,4-decadienoate |
| ethyl-2-methylbutyrate |
| ethylene brassylate (CAS No. 105-95-3) |
| eucalyptol |
| eucalyptus oil |
| eugenol |
| exaltolide |
| excital |
| fenchone |
| Flor acetate |
| floral pyran (rosyrane super, CAS No. 60335-71-9) |
| Floralozone (3-(o-(and p-)Ethylphenyl)-2,2-dimethylpropionaldehyde) |
| FLORHYDRAL^{®} (3-(3-Isopropylphenyl)butanal) |
| fructone |
| frutene |
| furaneol |
| galaxolide |
| galbanum oil (CAS No. 8023-91-4) |
| galbascone, alpha- (CAS No. 56973-85-4) |
| GALBEX^{®} (galbanum specialty) |
| gamma dodecalactone |
| gamma methyl ionone |
| gamma undecalactone |
| gamma-Decalactone |
| gamma-Nonalactone |
| garlic Oil |
| geosmin Neat |
| geraniol and ester derivatives |
| geranium oil |
| geranyl nitrile |
| ginger extract |
| glycolic acid, 2-pentyloxy-, allyl ester |
| grapefruit Zest (C&A) |
| hedione |
| helichrysum oil (CAS No. 90045-56-0) |
| helional |
| heliotropin |
| heptanal |
| hexadecanolide (juniper lactone, CAS No. 109-29-5) |
| hexanoic acid, 2-propenyl ester |
| hexenol |
| hexyl acetate |
| hexyl cinnamic aldehyde |
| hexyl salicylate |
| hivernal mixture |
| hydroxycitrolnellal |
| IH-indene-a-propanal |
| immortelle absolute (CAS No. 977060-66-4) |
| indole (CAS No. 120-72-9) |
| intreleven aldehyde |
| ionone |
| ionone beta |
| ionone gamma methyl |
| ionone, alpha- (CAS No. 127-41-3) |
| ionones |
| irisantheme |
| irone |
| irones, extract |
| Iso 2-Methoxy-4-(2-propenyl)phenol |
| iso bornyl acetate |
| iso cyclo citral |
| Iso E super (1,2,3,4,5,6,7,8-Octahydro-1,1,6,7-Tetramethyl-7-Acetyl Naphthalene) |
| iso propyl quinoline |
| isoamyl acetate |
| isoamyl iso-valerate |
| Isobomyl acetate |
| isobornyl acetate (1,7,7-trimethyl-bicyclo[2.2.1]hept-2-yl acetate) |
| isobutyl acetate |
| isobutyl quinoline |
| iso-butyl-(z)-2-methyl-2-butenoate |
| isobutylthiazole |
| isocyclocitral |
| isoeugenol |
| ISOPAR^{™} M (Isoparaffinic Hydrocarbon distillate) |
| isopropyl Myristate (3,7-Dimethyl-1,6-Octadien-3-Ol) |
| isovaleric acid (CAS No. 503-74-2) |
| isovaleric aldehyde |
| isovaleric aldehyde 0.1% DPG |
| jasmin absolute (Egypt, India, Maroc, or Sambac) |
| jasmine extract |
| JAVANOL^{®} (sandal cyclopropane, CAS No. 198404-98-7) |
| Jonquille absolute |
| karanal |
| ketone, methyl-2,6,10-trimethyl-2,5,9-cyclododecatriene-1-yl |
| KHARISMAL^{®} (methyl dihydorjasmonate; CAS No. 24851-98-7) |
| khusinil (grapefruit nitrile, CAS No. 75490-39-0) |
| KOUMALACTONE^{®} (dihydromint lactone, CAS No. 92015-65-1) |
| labdanum absolute (Ciste absolute, CAS No. 8016-26-0) |
| labdanum oil |
| labienoxime (*e.g.*, 10% labienone oxim in DPG) |
| lactone of cis-jasmone |
| lauronitrile |
| lavandin |
| lavender oil |
| L-carvone |
| lemon juice carbonyls |
| lemon oil |
| LEMONILE^{®} (homogeranyl nitrile, CAS No. 61792-11-8) |
| liffarome |
| lilial |
| liminal |
| limonene (1-Methyl-4-(1-methylethenyl)-cyclohexene) |
| limoxal (limonene oxo aldehyde) |
| linalool (3,7-dimethyl-1,6-octadien-3-ol) |
| linalyl acetate |
| L-menthol |
| lovage oil (essential, root, leaf) |
| lyral |
| majantol |
| maltol (2-methyl-3-hydroxypyrone, palatone, CAS No. 118-71-8) |
| mandarin aldehyde |
| mandarin oil |
| manzanate |
| maple lactone |
| mayol |
| menthe oil pays |
| menthol |
| menthone |
| methyl 2,4-dihydroxy-3,6-dimethylbenzoate (VERAMOSS^{®}, CAS No. 4707-47-5) |
| methyl 2-aminobenzoate |
| methyl 2-nonynonate |
| methyl 2-octynoate |
| methyl 2-pyridyl ketone |
| methyl anthranilate |
| methyl benzoate |
| methyl beta naphthyl ketone |
| methyl cedrenyl ketone |
| methyl cedrylone |
| methyl cinnamate CAS No. 103-26-4) |
| methyl dihydrojasmonate (CAS No. 24851-98-7) |
| methyl eugenol |
| methyl heptine carbonate |
| methyl heptyl ketone (2-nonanone, CAS No. 821-55-6) |
| methyl ionone (Xandralia) |
| methyl iso butenyl tetrahydro pyran |
| methyl jasmonate |
| methyl laitone |
| methyl nonyl acetaldehyde |
| methyl nonyl ketone |
| methyl octine carbonate |
| methyl pamplemousse |
| methyl phenyl carbinyl acetate |
| methyl quinoline Para |
| methyl salicylate |
| methyl tuberate (CAS No. 33673-62-0) |
| methyl-2-nonenoate |
| methyl-2-octynoate |
| methyl-3,4-dioxy(cylcoacetonyl) benzene |
| methylpyrazine |
| mimosa extract |
| mint oil crude arvensis |
| mugetanol |
| muscenone |
| musk ketone |
| musk oil |
| musk tibetine |
| musk xylol |
| myrrh resin |
| n-amyl acetate |
| n-amyl propionate |
| Naphtho(2,1-B)-furan,3A-Ethyl Dodecahydro-6,6,9A-Trimethyl |
| narcissus absolute (French, CO2) |
| narcissus extract |
| Natrotar Rectified BLO |
| Natural Sinensal |
| nectaryl (2-[2-(4-methyl-1-cyclohex-3-enyl)propyl]cyclopentan-1-one, CAS No. 95962-14-4) |
| nectrarol sa |
| neobutanone |
| neofolione |
| neononyl acetate (3,5,5-trimethylhexyl acetate) |
| nerol |
| neroli oil |
| NIRVANOL^{®} (3,3-dimethyl-5-(2,2,3-trimethyl-3-cyclopenten-l-yl)-4-penten- 2-ol |
| nona-2,6-dienenitrile, (2~{E},6~{Z})- |
| nonadienol, 2-trans-6-cis |
| nonanal |
| nonane diol-1,3-acetate |
| nonenal, cis-6 |
| nonenol, cis-6 |
| norlimbanol |
| nutmeg extract |
| oakmoss 25% |
| octahydro-2,2,5,8,8,9a-hexamethyl-4H-4a,9-methanoazuleno[5,6-d]-1,3-dioxole |
| octahydro-4,8a-dimethyl 4a(2H)-naphthol |
| octalactone gamma |
| octanal |
| olibanum extract |
| onion oil |
| orange CP |
| orange flower absolute (Morocco, Tunisia, extract, water absolute, etc.) |
| orange juice carbonyls |
| orange oil |
| orange sinensal |
| origanum oil |
| Orris absolute (pallida) |
| orris aldehyde (2-nonenal, CAS No. 18829-56-6) |
| ortho tertiary butyl cyclohexanyl acetate |
| orthotertiary-butyl cyclohexyl acetate |
| osmanthus absolute (CAS No. 68917-05-5) |
| o-tert-Butylcyclohexyl acetate (VERDOX^{®}) |
| Oxacyclohexadecen-2-one |
| Oxalone (CALONE^{®} 1951) |
| oxane ((2R,4S)-2-methyl-4-propyl-1,3-oxathiane, CAS No. 59323-76-1) |
| oxane, (2~{S},4~{R})-4-methyl-2-phenyl- |
| oxydibenzene |
| ozone propanal (Floralozone) |
| ozonil |
| P.t. bucinal |
| p-1-menthen-8 thiol |
| Para cresyl methyl ether |
| para hydroxy phenyl butanone |
| para-cresyl acetate (CAS No. 140-39-6) |
| Paradiff |
| para-iso-propyl alpha-octyl hydrocinnamic aldehyde |
| paramenthene |
| para-tertiary-butyl cyclohexyl acetate |
| patchouli oil |
| peppermint oil |
| PHARAONE^{®} (2-cyclohexyl-1,6-heptadien-3-one, CAS No. 313973-37-4) |
| phenethyl isobutyrate |
| phenol, 2-ethoxy-4-methyl- |
| phenol, 2-methoxy-4-prop-2-enyl- |
| phenoxyethyl isobutyrate |
| phenoxynol |
| phenyl acetaldehyde |
| phenyl ethyl acetate |
| phenyl ethyl alcohol |
| phenyl-acetaldehyde dimethyl acetate |
| pinenes |
| pinoacetaldehyde (CAS No. 33885-51-7) |
| pipol (distilled) |
| p-methylacetophenone |
| polysantol |
| prenyl acetate |
| propenyl guaethol (VANITROPE^{®}, CAS No. 94-86-0) |
| pyrazine, 2-butan-2-yl-3-methoxy- |
| pyrazine, 2-ethyl-3,5-dimethyl- |
| Pyrazine, 2-methoxy-3-(2-methylpropyl)- |
| pyrazines |
| pyrazobutyle |
| pyridine, 2-(1-ethylpropyl)- |
| pyridine, 2-pentan-3-yl- |
| pyridine, 4-[(3~{E})-4,8-dimethylnona-3,7-dienyl]- |
| Quinoline, 6-secondary butyl |
| RHUBOFIX^{®} (rhubarb oxirane; CAS No. 41816-03-9) |
| robustone |
| romanolide |
| ROMASCONE^{®} (damascene carboxylate) |
| rose absolute (Damascena pure, Maroc, MD, Turkish, Vah Dijon, etc.) |
| rose centifolia absolute |
| rose essential |
| rose extract |
| rose oil (Bulgarian, Isparta, Turkish, etc.) |
| rose oxide |
| rosemary oil |
| SAFRALEINE^{®} (2,3,3-trimethyl-2H-inden-1-one, CAS No. 54440-17-4) |
| safranal (CAS No. 116-26-7) |
| sage oil |
| SANDALORE^{®} (Sandal pentanol; CAS No. 65113-99-7) |
| sandalwood oil |
| sanjinol |
| santalol |
| schinus molle absolute |
| schinus molle CO2 extract |
| schinus molle oil |
| spearmint oil |
| spiro[furan-2(3H),5'[4,7]methanol[5H]indene], decahydro- |
| spiro[oxolane-2,8'-tricyclo[5.2.1.0^{2,6}]decane] |
| spirogalbone |
| STARFLEUR^{®} (3-methyl-4-phenylbutan-2-ol, CAS 56836-93-2) |
| STEMONE^{®} (leafy oxime; CAS No. 22457-23-4) |
| sulfurol |
| tagette oil (Egypt, MD, etc.) |
| tangerinal |
| tarragon oil |
| terpenes |
| terpinolene |
| tetra Hydro 3,7-Dimethyl-1,6-octadien-3-ol |
| tetradecanoic acid |
| tetrahydrolinalool |
| tetrahydromuguol |
| thiazole (4-iso propyl 2-methyl) |
| thienopyrimidine |
| thymol |
| thymol oil |
| tonalide |
| trans-2-decenal |
| trans-2-dodecenal |
| trans-2-hexenal |
| trans-4-decenal |
| trans-anethole |
| trans-Pinoacetaldehyde |
| trichloro methyl phenyl carbinyl acetate |
| Tricyclo decenyl acetate |
| tricyclo[5.2.1.02,6]decane-3-carbaldehyde (MELOZONE^{®}, CAS No. 30772-79-3) |
| trideca-3,12-dienenitrile, (3~{E})- |
| tridecene-2-nitrile |
| trifemal |
| trimofix O (Amber Decatriene) |
| TRIPLAL^{®} (2,4-ivy carbaldehyde; CAS No: 68039-49-6) |
| tripropylene Glycol monomethyl ether ((2-(2-Methoxymethylethoxy)methylethoxy)propanol |
| tuberose extract |
| undecalactone |
| undecanal |
| undecavertol |
| undeclactone |
| undecyl aldehyde |
| undecylenic aldehyde |
| vanilla (absolute) |
| vanillin |
| verbena oil |
| verdoracine (1-methyl-4-propan-2-yl-2-prop-1-enylbenzene, CAS No. 14374-92-6) |
| VERDOX^{®} (2-t-butyl cyclohexyl acetate) |
| verdural B extra ((Z)-3-hexen-1-yl isobutyrate, CAS No. 41519-23-7) |
| Vertenex |
| Vertoliff |
| Vetiver oil |
| vetyver |
| violet leaf absolute |
| violiff |
| Vivaldie |
| wintergreen oil |
| yara yara oil |
| ylang extract |
| ylanganate (methyl 2-methyl benzoate, CAS No. 89-71-4) |
| zestoril (CAS No. 1208985-45-8) |
| zestover (2,4-Dimethyl-3-Cyclohexene-1-Carboxaldehyde) |
| β-caryophylene (CAS No. 87-44-5) |

Preferred High-Performance fragrance ingredients for use in the fragrance composition of this invention are shown in Table 2 below.

**TABLE 2**

| Fragrance | Chemical Name | CAS No. |
|---|---|---|
| Abhexone | 2~{H}-furan-5-one, 2-ethyl-4-hydroxy-3-methyl- | 698-10-2 |
| AGRUNITRILE | 6-octenenitrile, 3,7-dimethyl- | 51566-62-2 |
| Amberketal 8.5 Pct IPM | 14,16-dioxatetracyclo[11.2.1.0^{1,10}.0^{4,9}]hexade cane, 5,5,9,13-tetramethyl- | 57345-19-4 |
| Amber Xtreme (ELINCS) | 3,3a,4,5,5a,7,8a,8b-octahydrocyclopenta[g]benzofuran, (3aS,8bS)-2,2,6,6,7,8,8-heptamethyl- | 476332-65-7 |
| AMBRINOL 95 PRG | 1,3,4,4~{a},6,7-hexahydronaphthalen-2-ol, 2,5,5-trimethyl- | 41199-19-3 |
| ALD C-16 STRAWB#2 | 2-oxiranecarboxylic acid, (2S,3R)-3-methyl-3-phenyl-, ethyl ester | 19464-92-7 |
| ACETOPHENONE | ethanone, 1-phenyl- | 98-86-2 |
| Acetyl 2-, Pyridine 10% IPM | ethanone, 1-(2-pyridinyl)- | 1122-62-9 |
| ALD AA TRIPLAL BHT | 1-cyclohex-3-enecarboxaldehyde, (1S,2R)-2,4-dimethyl- | 188716-52-1 |
| Ald C-6 Toco | hexanal | 66-25-1 |
| ALD C-8 TOCO | octanal | 124-13-0 |
| ALD C-10 | decanal | 112-31-2 |
| ALD C-9 TOCO | nonanal | 124-19-6 |
| ALD C-11 ULENIC TOCO | 10-undecenal | 112-45-8 |
| ALD C-11 UNDECYLIC TOCO | undecanal | 112-44-7 |
| ALD C-12 LAURIC TOCO | dodecanal | 112-54-9 |
| ALD C-18 | 2-oxolanone, 5-pentyl- | 104-61-0 |
| MELOZONE | 3-tricyclo[5.2.1.02,6]decanecarboxaldehyde, (1S,2R,6S,7R)- | 30772-79-3, 91967-77-0 |
| AMBROCENIDE CRYST | 4,6-dioxatetracyclo[6.5.1.01,10.03,7]tetradecane, 5,5,7,9,9,13-hexamethyl- | 211299-54-6 |
| AMBRETTOLIDE | 1-oxacycloheptadec-10-en-2-one, (10~{E})- | 63286-42-0 |
| AMBERMOR EX | 2,4,5,5~{a},7,8,9,9~{b}-octahydro-1~{H}-benzo[e]benzofuran, (3~{a}~{S},5~{a}~{R},9~{a}~{R},9~{b}~{S})-3~{a},6,6,9~{a}-tetramethyl- | 3738-00-9, 6790-58-5 |
| ACETYL METH CARBINOL 10% DPG | 2-butanone, 3-hydroxy- | 513-86-0 |
| BENZALD FFC 10% DPG | benzaldehyde | 100-52-7 |
| CALONE | 1,5-benzodioxepin-3-one, 7-methyl- | 28940-11-6 |
| CASSIS ETHER IPM | tetradecanoic acid, propan-2-yl ester | 110-27-0 |
| CASHMERAN | 2,5,6,7-tetrahydroinden-4-one, 1,1,2,3,3-pentamethyl- | 33704-61-9 |
| Corps Pamplemousse Pure | 2-propanethiol, 2-(4-methyl-1-cyclohexenyl)- | 61758-19-8 |
| CYCLEMAX | propanal, 3-(4-propan-2-ylphenyl)- | 7775-00-0 |
| CEDRAMBER | tricyclo[5.3.1.0^{1,5}]undecane, (1~{S},2~{R},5~{S},8~{R})-8-methoxy-2,6,6,8-tetramethyl- | 67874-81-1 |
| CITRAL NEW | octa-2,6-dienal, (2E)-3,7-dimethyl- | 141-27-5 |
| Citrylal | benzene, 1-methyl-4-propan-2-yl- | 99-87-6 |
| Clonal | dodecanenitrile | 2437-25-4 |
| Corps Racine Vs 600164 Conc | pyridine, 2-(3-phenylpropyl)- | 2110-18-1 |
| Costasid | 2-octenoic acid, 4-ethyl- | 90464-78-1 |
| CREOSOL #340 | phenol, 2-methoxy-4-methyl- | 93-51-6 |
| CYCLAPROP | propanoic acid, 8-tricyclo[5.2.1.0^{2,6}]dec-4-enyl ester | 67634-24-6 |
| Dimethyl Sulfide Nat | methane, (methylthio)- | 75-18-3 |
| Decenal, 9, | 9-decenal | 39770-05-3 |
| Dimeth Phenol,2,3, | phenol, 2,3-dimethyl- | 526-75-0 |
| Dimeth Phenol,2,4, | phenol, 2,4-dimethyl- | 105-67-9 |
| DORIFFOX | oxane, (2~{S},4~{R})-4-methyl-2-phenyl- | 149713-23-5, 149713-24-6, 94201-73-7 |
| DODECENAL TRANS-2 TOCO | 2-dodecenal, (~{E})- | 20407-84-5 |
| OPALENE BHT | 8-decenal, (~{E})- | 174155-47-6 |
| DECENAL,2, TOCO | 2-decenal, (~{E})- | 3913-81-3 |
| DECENAL,CIS-4 | 4-decenal, (~{Z})- | 21662-09-9 |
| DELPHONE | 1-cyclopentanone, 2-pentyl- | 4819-67-4 |
| DAMASCONE DELTA | 2-buten-1-one, (E)-1-[(1S,2R)-2,6,6-trimethyl-1-cyclohex-3-enyl]- | 71048-82-3 |
| DIPHEN OXIDE | benzene, phenoxy- | 101-84-8 |
| DUPICAL NAAR | butanal, 4-(8-tricyclo[5.2.1.02,6]decanylidene)- | 30168-23-1 |
| EUGENOL NAT EX CLOVE LEAF OIL | phenol, 2-methoxy-4-prop-2-enyl- | 97-53-0 |
| TROPICALIA | 2-bicyclo[2.2.1]hept-5-enecarboxylic acid, (2~{R})-, ethyl ester | 51789-95-8 |
| ETH ACETO ACET 10% DPG | butanoic acid, 3-oxo-, ethyl ester | 141-97-9 |
| ETH CAPROATE | hexanoic acid, ethyl ester | 123-66-0 |
| ETH-2-METH BUTY | butanoic acid, 2-methyl-, ethyl ester | 7452-79-1 |
| ETH PHEN GLYC | 2-oxiranecarboxylic acid, (2~{S},3~{R})-3-phenyl-, ethyl ester | 2272-55-1 |
| ETH SAL | benzoic acid, 2-hydroxy-, ethyl ester | 118-61-6 |
| EUCALYPTOL USP | 2-oxabicyclo[2.2.2]octane, 1,3,3-trimethyl- | 470-82-6 |
| FILBERTONE 22350 | 2-hepten-4-one, (~{E})-5-methyl- | 102322-83-8 |
| STARFLEUR TOCO | propanal, 3-[4-(2-methylpropyl)cyclohexyl]- | 1315250-65-7 |
| FRUITATE (ELINCS) | 2-tricyclo[5.2.1.0^{2,6}]decanecarboxylic acid, ethyl ester | 129520-41-8 |
| FLORAL SUPER | deca-4,9-dienal, (4E)-4,8-dimethyl- | 77016-39-8 |
| FLORHYDRAL TOCO (ELINCS) | butanal, 3-(3-propan-2-ylphenyl)- | 125109-85-5 |
| FRAGARONE | 2-pentenoic acid, (~{E})-2-methyl- | 16957-70-3 |
| Galbazine | pyrazine, 2-methoxy-3-(2-methylpropyl)- | 24683-00-9 |
| Geosmin Neat | 1,2,3,4,5,6,7,8-octahydronaphthalen-4~{a}-ol, 4,8~{a}-dimethyl- | 19700-21-1 |
| GALBASCONE | 4-penten-1-one, 1-(5,5-dimethyl-1-cyclohexenyl)- | 56973-85-4 |
| Ald C-7 Stabiliff | heptanal | 111-71-7 |
| HEALINGWOOD | | 5986-55-0 |
| METH DH JASMONATE | acetic acid, 2-[(1~{S},2~{S})-3-oxo-2-pentylcyclopentyl]-, methyl ester | 133163-97-0, 2570-03-8, 29852-02-6 |
| HEXADECANOLIDE | oxacycloheptadecan-2-one | 109-29-5 |
| Homo Pineapple CMPD | 3-furanone, 2-ethyl-4-hydroxy-5-methyl- | 27538-10-9 |
| ISO VALERIC ACID 0.1% DPG | butanoic acid, 3-methyl- | 503-74-2 |
| ALD C-11 INTRELEVEN (TT) PRG | 9-undecenal, (~{E})- | 143-14-6 |
| IRONE V BHT 1% DPG | 3-buten-2-one, (~{E})-4-[(1~{S},5~{R})-2,5,6,6-tetramethyl-1-cyclohex-2-enyl]- | 79-69-6 |
| Thiazole (4-Iso Propyl 2-Methyl) | thiazole, 4-methyl-2-propan-2-yl- | 15679-13-7 |
| IONONE ALPHA BHT | 3-buten-2-one, (~{E})-4-(2,6,6-trimethyl-1-cyclohex-2-enyl)- | 31798-11-5 |
| ORRIS ALD | 2-nonenal, (~{E})- | 18829-56-6 |
| ISO BUTYL QUINOLINE | quinoline, 6-butan-2-yl- | 65442-31-1 |
| ISO CYCLO CITRAL BHT | 1-cyclohex-3-enecarboxaldehyde, (1~{R},2~{S},6~{S})-2,4,6-trimethyl- | 1423-46-7 |
| Iso Propyl Quinoline | quinoline, 6-propan-2-yl- | 135-79-5 |
| Iso Valeric Ald | butanal, 3-methyl- | 590-86-3 |
| JAVANOL TT (ELINCS) | methanol, [1-methyl-2-[(1,2,2-trimethyl-3-bicyclo[3.1.0]hexanyl)methyl]cyclopropyl]- | 198404-98-7 |
| KOUMALACTONE 10 PCT TEC FIRM | 3a,4,5,6,7,7a-hexahydro-3H-benzofuran-2-one, 3,6-dimethyl- | 79726-51-5, 92015-65-1 |
| KHUSINIL (ELINCS) | pentanenitrile, 2,2-dimethyl-4-phenyl- | 75490-39-0 |
| LACTONE OF CIS JASMONE TOCO | 2-oxolanone, 5-[(~{Z})-hex-3-enyl]-5-methyl- | 70851-61-5 |
| Limoxal (Limonene Oxo Ald) | butanal, 3-(4-methyl-1-cyclohex-3-enyl)- | 50450-53-8, 6784-13-0 |
| LEMONILE | nona-2,6-dienenitrile, (2~{Z},6~{Z})-3,7-dimethyl- | 61792-11-8 |
| Undecatriene Super LRG 1218 RD BHT | undeca-1,3,5-triene, (3~{E},5~{E})- | 16356-11-9 |
| METH PAMPLEMOUSSE TOCO | 2-hexene, 6,6-dimethoxy-2,5,5-trimethyl- | 67674-46-8 |
| Maritima | pyridine, 4-[(3~{E})-4,8-dimethylnona-3,7-dienyl]- | 69511-23-5 |
| MANDARIL (ELINCS) BHT | trideca-3,12-dienenitrile, (3~{E})- | 134769-33-8 |
| MONTAVERDI | cyclopropanecarboxylic acid, [(~{Z})-hex-3-enyl] ester | 16428-99-2, 188570-78-7 |
| MUSCEMOR (ELINCS) | 1-cyclopentadec-5-enone, 3-methyl- | 63314-79-4 |
| METH JASMONATE TOCO | acetic acid, 2-[(1~{R},2~{S})-3-oxo-2-[(~{E})-pent-2-enyl]cyclopentyl]-, methyl ester | 95722-42-2 |
| MENTHONE 85 | 1-cyclohexanone, (2S,5R)-5-methyl-2-propan-2-yl- | 1074-95-9, 14073-97-3, 89-80-5 |
| METH LAITONE 10 PCT DPG | 1-oxaspiro[4.5]decan-2-one, 8-methyl- | 94201-19-1 |
| VERIDIAN | 3-decen-5-one, (~{E})-4-methyl- | 811412-48-3 |
| METH BENZOATE | benzoic acid, methyl ester | 93-58-3 |
| METH BUTYRIC ACID,2, | butanoic acid, 2-methyl- | 116-53-0 |
| METH CINNAMATE TOCO | 2-propenoic acid, (~{E})-3-phenyl-, methyl ester | 1754-62-7 |
| Jamunate | 1-cyclohexanecarboxylic acid, 3-methyl-, methyl ester | 72903-23-2 |
| MELONAL TOCO | 5-heptenal, 2,6-dimethyl- | 106-72-9, 77787-60-1 |
| Meth Heptin Carbonate | 2-octynoic acid, methyl ester | 111-12-6 |
| METH HEPTYL KETONE | 2-nonanone | 821-55-6 |
| ALD C-12 MNA TOCO | undecanal, 2-methyl- | 110-41-8 |
| METH OCTIN CARBONATE | 2-nonynoic acid, methyl ester | 111-80-8 |
| ALD C-11 MOA BHT | decanal, 2-methyl- | 19009-56-4 |
| METH PHEN ETH ETHER | benzene, 2-methoxyethyl- | 3558-60-9 |
| METH TUBERATE RD | 2-oxolanone, 4-methyl-5-pentyl- | 33673-62-0 |
| MANGONE | 1-cyclohexanone, (2~{S},5~{S})-2-(2-mercaptopropan-2-yl)-5-methyl- | 33281-91-3 |
| CRISTALFIZZ | butanal, 4-heptoxy-3-methyl- | 1093653-57-6 |
| AQUAFLORA TOCO | acetaldehyde, 2-[(1S,2R,6R,7S,8R)-8-tricyclo[5.2.1.02,6]decanyl]- | 1339119-15-1 |
| Pomelene 0.01Pct Ipm | 6-thiabicyclo[3.2.1]octane, 4,7,7-trimethyl- | 68398-18-5 |
| METH PARA CRESOL | benzene, 1-methoxy-4-methyl- | 104-93-8 |
| MUSCENONE (ELINCS) | 1-cyclopentadec-5-enone, 3-methyl- | 63314-79-4 |
| MYRAC ALD BHT | 1-cyclohex-3-enecarboxaldehyde, 4-(4-methylpent-3-enyl)- | 37677-14-8 |
| VERTONIC FOR NON TSCA USE ONLY | 3,6-dihydro-2~{H}-pyran, 4,6-dimethyl-2-(1-phenylethyl)- | 1945993-03-2 |
| NECTAROL SA | 1-cyclohex-2-enone, 3,5-diethyl-2,5-dimethyl- | 39121-42-1 |
| NECTARYL LRG | 1-cyclopentanone, 2-[2-(4-methyl-1-cyclohex-3-enyl)propyl]- | 95962-14-4 |
| Nonenol,Cis-6 Toco | 6-nonen-1-ol, (~{Z})- | 35854-86-5 |
| NONADIENAL 2-6 DIETH ACETAL | nona-2,6-diene, (2~{E},6~{Z})-1,1-diethoxy- | 67674-36-6 |
| Nonenal,Cis-6 Toco | 6-nonenal, (~{Z})- | 2277-19-2 |
| OXANE 50 PCT TEC | 1,3-oxathiane, (2~{R},4~{S})-2-methyl-4-propyl- | 59323-76-1 |
| OPERANIDE (ELINCS) | 4,5,7,8b-tetrahydro-3aH-cyclopenta[e][1,3]benzodioxole, 2,2,6,6,7,8,8-heptamethyl- | 823178-41-2 |
| ORENYLE | 2-nonenenitrile, (~{E})- | 40856-16-4 |
| Pyrazine,2-Eth-3,5-Dimeth | pyrazine, 2-ethyl-3,5-dimethyl- | 13925-07-0 |
| Pharaone 10 PCT DPG | 3-hepta-1,6-dienone, 2-cyclohexyl- | 313973-37-4 |
| MALTOL (PALATONE) | 4-pyranone, 3-hydroxy-2-methyl- | 118-71-8 |
| CRESETAL PARA | oxane, 2-(4-methylphenoxy)- | 13481-09-9 |
| CRESOL PARA EXTRA | phenol, 4-methyl- | 106-44-5, 1319-77-3 |
| Cresyl Acet Para Coeur | acetic acid, (4-methylphenyl) ester | 140-39-6 |
| Meth Quinoline Para | quinoline, 6-methyl- | 91-62-3 |
| PASSION FRUIT COMPOUND | | |
| PINEAPPLE CMPD 1% IPM (MB) | 3-furanone, 4-hydroxy-2,5-dimethyl- | 3658-77-3 |
| PATCHOULI OIL LIGHT BLO | 3-tricyclo[5.3.1.03,8]undecanol, 2,2,6,8-tetramethyl- | 5986-55-0 |
| PENTYL FURAN,2, | furan, 2-pentyl- | 3777-69-3 |
| Phenacetic Acid Extra (USDEA) | acetic acid, 2-phenyl- | 103-82-2 |
| PHEN ETH ACET | acetic acid, 2-phenylethyl ester | 103-45-7 |
| PINO ACETALD TOCO | propanal, 3-(6,6-dimethyl-2-bicyclo[3.1.1]hept-2-enyl)- | 33885-51-7 |
| POPCORN CHEMICAL | 5,7-dihydrothieno[3,4-d]pyrimidine, 2-methyl- | 36267-71-7 |
| PYRAZINE PM517 2-B-3-MEOXY | pyrazine, 2-butan-2-yl-3-methoxy- | 24168-70-5 |
| ROSYRANE SUPER | oxane, 4-methylene-2-phenyl- | 60335-74-2 |
| Robustone | furan, 2-[(2-furanylmethyldisulfanyl)methyl]- | 4437-20-1 |
| ROSALVA | 9-decen-1-ol | 13019-22-2 |
| ROSE OXIDE TOCO | oxane, (2~{R},4~{S})-4-methyl-2-(2-methylprop-1-enyl)- | 3033-23-6 |
| ROSETHYL | benzene, 1-(ethoxymethyl)-2-methoxy- | 64988-06-3 |
| Safranal Toco | 1-cyclohexa-1,3-dienecarboxaldehyde, 2,6,6-trimethyl- | 116-26-7 |
| SINENSAL NATURAL 20 EX ORANGE | dodeca-2,6,11-trienal, (2E,6E)-2,6-dimethyl-10-methylene- | 3779-62-2 |
| OCEANOL | benzoic acid, 2,4-dihydroxy-3-methyl-, methyl ester | 33662-58-7 |
| SAFRALEINE | 2~{H}-inden-1-one, 2,3,3-trimethyl- | 54440-17-4 |
| Spirogalbanone 10% DEP | 4-penten-1-one, 1-(9-spiro[4.5]dec-9-enyl)- | 224031-71-4 |
| Sacrazole-018 | ethanol, 2-(4-methyl-5-thiazolyl)- | 137-00-8 |
| SINFONIDE | 6,6a,8,9a-tetrahydro-5H-cyclopenta[h]quinazoline, (6aR,9aR)-7,7,8,9,9-pentamethyl- | 1356400-59-3 |
| TOFFEE LACTONE 2067 | 2-oxolanone, 5-hexyl- | 107797-26-2, 706-14-9 |
| TRIFERNAL BHT 10% DPG | butanal, 3-phenyl- | 16251-77-7 |
| COOLWOOD | 8-tricyclo[5.2.1.02,6]decanol, 5,7,8-trimethyl- | 1340502-69-3 |
| TERPINOLENE P UB BHT | cyclohexene, 1-methyl-4-propan-2-ylidene- | 586-62-9 |
| Pyrazine 004 (2-Ethyl-3-Meth) | pyrazine, 2-ethyl-3-methyl- | 15707-23-0 |
| Pyrazine 044 (Methyl) | pyrazine, 2-methyl- | 109-08-0 |
| Pyrazine 068 (2,6-Dimeth) | pyrazine, 2,6-dimethyl- | 108-50-9 |
| Pyrazine 043 (2,5-Dimeth) | pyrazine, 2,5-dimethyl- | 123-32-0 |
| Pyrazine 040 (2,3-Dimeth) | pyrazine, 2,3-dimethyl- | 5910-89-4 |
| Pyrazine 001 (2,3,5-Trimeth) | pyrazine, 2,3,5-trimethyl- | 14667-55-1 |
| Pyrazine 014 (Cyclo Hexa) | 5,6,7,8-tetrahydroquinoxaline | 34413-35-9 |
| Iso Butyl Thiazole | thiazole, 2-(2-methylpropyl)- | 18640-74-9 |
| TONKALACTONE | 3a,4,5,6,7,7a-hexahydro-3H-benzofuran-2-one, 3-ethyl- | 54491-17-7 |
| NONADIENAL,2-TR-6-CIS TOCO | nona-2,6-dienal, (2~{E},6~{Z})- | 557-48-2 |
| Nonadienol,2-Trans-6-Cis Toco | 1-nona-2,6-dienol, (2~{E},6~{Z})- | 28069-72-9 |
| DECENAL,TRANS 4, TOCO | 4-decenal, (~{E})- | 65405-70-1 |
| HEXENAL,TRANS 2, | 2-hexenal, (~{E})- | 6728-26-3 |
| Tridecene-1-Al,2, Toco | 2-tridecenal, (~{E})- | 7069-41-2 |
| Tridecene-2-nitrile | 2-tridecenenitrile, (~{E})- | 22629-49-8 |
| UNDECALACTONE,D ELTA | 2-oxanone, 6-hexyl- | 710-04-3 |
| ULTRAVANIL Q COLIPA | phenol, 2-ethoxy-4-methyl- | 2563-07-7 |
| UNDECAVERTOL TOCO | 3-decen-5-ol, (~{E})-4-methyl- | 81782-77-6 |
| VANITROPE | phenol, 2-ethoxy-5-[(~{E})-prop-1-enyl]- | 94-86-0 |
| VIONIL NEAT | nona-2,6-dienenitrile, (2~{E},6~{Z})- | 97752-28-8 |
| VARAMOL-106 | phenol, 4-ethenyl-2-methoxy- | 7786-61-0 |
| VELTOL PLUS | 4-pyranone, 2-ethyl-3-hydroxy- | 4940-11-8 |
| VERAMOSS | benzoic acid, 2,4-dihydroxy-3,6-dimethyl-, methyl ester | 4707-47-5 |
| Verdima | pyridine, 2-pentan-3-yl- | 7399-50-0 |
| VERDURAL B EXTRA | propanoic acid, 2-methyl-, [(~{Z})-hex-3-enyl] ester | 41519-23-7 |
| Verdoracine | benzene, 1-methyl-4-propan-2-yl-2-[(~{E})-prop-1-enyl]- | 14374-92-6 |
| VETIVERT ACET HAITI BLO | acetic acid, [(1~{R},5~{S})-7,7-dimethyl-6-methylene-2-tricyclo[6.2.1.0^{1,5}]undecanyl]methyl ester | 52771-09-2 |
| VIGOFLOR | spiro[oxolane-2,8'-tricyclo[5.2.1.0^{2,6}]decane] | 68480-11-5 |
| YLANGANATE | benzoic acid, 2-methyl-, methyl ester | 89-71-4 |
| YARA YARA | naphthalene, 2-methoxy- | 93-04-9 |
| Zestoril | 2-propanethiol, 2-(4-methyl-1-cyclohexenyl)- | 61758-19-8 |
| ANGELICA ROOT OIL | Natural oil, no chemical name | |
| ARMOISE OIL PURE | Natural oil, no chemical name | |
| ANISE SEED OIL SPANISH | Natural oil, no chemical name | |
| ANGELICA SEED OIL | Natural oil, no chemical name | |
| ARMOISE ESS ROBT | Natural oil, no chemical name | |
| ASAFETIDA ROOT OIL | Natural oil, no chemical name | |
| BASIL OIL GRAND VERT LMR | Natural oil, no chemical name | |
| BASIL OIL VERBENA LMR | Natural oil, no chemical name | |
| BASIL OIL VIETNAM LMR | Natural oil, no chemical name | |
| BLKCURNT BUD ABS LMR FLG FOR LIFE | Natural oil, no chemical name | |
| BASIL OIL SWEET | Natural oil, no chemical name | |
| BASIL ABS GRAND VERT LMR | Natural oil, no chemical name | |
| BASIL ABS GRAND VERT MD LMR | Natural oil, no chemical name | |
| BIRCH TAR RECT EXTRA | Natural oil, no chemical name | |
| BASIL OIL | Natural oil, no chemical name | |
| BUCHU LEAF OIL BETULINA | Natural oil, no chemical name | |
| Blackcurrant Bud Abs | Natural oil, no chemical name | |
| BUCHU LEAF OIL BETULINA | Natural oil, no chemical name | |
| CADE OIL RECT | Natural oil, no chemical name | |
| CHAMOMILE OIL ROMAN LMR SFO | Natural oil, no chemical name | |
| CINNAMON BARK OIL CEYLON LMR | Natural oil, no chemical name | |
| COFFEE OIL FILT BRAZ. | Natural oil, no chemical name | |
| CARDAMOM OIL GUATEMALA | Natural oil, no chemical name | |
| CYPRIOL | Natural oil, no chemical name | |
| CARDAMOM GUAT EXTRACT CO2 LMR | Natural oil, no chemical name | |
| CARDAMOM OIL GUATEMALA LMR | Natural oil, no chemical name | |
| CARDAMOM OIL EI LMR | Natural oil, no chemical name | |
| CHAMOMILE OIL ENG | Natural oil, no chemical name | |
| CASTOREUM RESOID LMR | Natural oil, no chemical name | |
| CISTE ABS LMR | Natural oil, no chemical name | |
| CHAMOMILE OIL WILD LMR | Natural oil, no chemical name | |
| CISTE ABS C'LESS LMR | Natural oil, no chemical name | |
| COFFEE ABS ARABICA CO2 | Natural oil, no chemical name | |
| CHAMOMILE OIL WILD LOW LIM REF A LMR | Natural oil, no chemical name | |
| CYPRIOL OIL LMR | Natural oil, no chemical name | |
| CYPRIOL HEART LMR | Natural oil, no chemical name | |
| CINNAMON BARK ESSENTIAL LMR | Natural oil, no chemical name | |
| CUMIN SEED OIL PURE | Natural oil, no chemical name | |
| DAVANA OIL LMR FLG SFO | Natural oil, no chemical name | |
| DYNAMONE SB | Natural oil, no chemical name | |
| EUCALYPTUS OIL 80/85 NP | Natural oil, no chemical name | |
| TARRAGON OIL PURE | Natural oil, no chemical name | |
| EUCALYPTUS OIL 80/85 TOCO | Natural oil, no chemical name | |
| FIR BALSAM ABS | Natural oil, no chemical name | |
| FIR BALSAM ABS RESIN | Natural oil, no chemical name | |
| Garlic Oil NAT EGYPT 1% DEP | Natural oil, no chemical name | |
| GALBANOL LMR | Natural oil, no chemical name | |
| GALBANUM OIL LMR | Natural oil, no chemical name | |
| GALBANUM RESOID LMR | Natural oil, no chemical name | |
| GALBANUM HEART LMR | Natural oil, no chemical name | |
| HELICHRYSUM OIL | Natural oil, no chemical name | |
| HYDRO CARBON RESIN SB | Natural oil, no chemical name | |
| IRONES EX ORRIS TECH LMR | Natural oil, no chemical name | |
| IMMORTELLE ABS BALKANS LMR | Natural oil, no chemical name | |
| JASMIN ABS INDIA MD LMR | Natural oil, no chemical name | |
| JASMIN ABS MAROC LMR | Natural oil, no chemical name | |
| JASMIN ABS SAMBAC MD LMR | Natural oil, no chemical name | |
| JASMIN ABS INDIA LMR | Natural oil, no chemical name | |
| JONQUILLE ABS FRANCE LMR | Natural oil, no chemical name | |
| JASMIN ABS SAMBAC LMR | Natural oil, no chemical name | |
| JASMIN ABS EGYPT LMR | Natural oil, no chemical name | |
| CISTE ABS SIS F0785 | Natural oil, no chemical name | |
| Leatherwood LMR XBOX | Natural oil, no chemical name | |
| LOVAGE OIL | Natural oil, no chemical name | |
| NARCISSE ABS FRENCH LMR | Natural oil, no chemical name | |
| Natrotar Rect BLO | Natural oil, no chemical name | |
| NARCISSE ABS FRENCH CO2 LMR | Natural oil, no chemical name | |
| ORANGE FLOWER ABS MOROCCO LMR | Natural oil, no chemical name | |
| ORANGE FLOWER WATER ABS TUNISIA LMR | Natural oil, no chemical name | |
| ORANGE FLOWER ABS TUNISIA LMR | Natural oil, no chemical name | |
| OSMANTHUS ABS LMR | Natural oil, no chemical name | |
| ORIGANUM OIL TURKISH | Natural oil, no chemical name | |
| ORRIS RESOID LMR | Natural oil, no chemical name | |
| ORRIS CRET MOROCCO FOR LMR | Natural oil, no chemical name | |
| ORRIS MOROC NAT 15PCT 4117C LMR | Natural oil, no chemical name | |
| ORRIS CRET 8 PCT IRONE LMR | Natural oil, no chemical name | |
| ORRIS ABS PALLIDA LMR | Natural oil, no chemical name | |
| Onion Oil NAT 1% ETOH | Natural oil, no chemical name | |
| ORANGE FLOWER ABS BLO | Natural oil, no chemical name | |
| PEPPER PINK CO2 LMR | Natural oil, no chemical name | |
| PATCHOULI OIL LIGHT BLO | Natural oil, no chemical name | |
| PEPPERMINT NAT SLCT | Natural oil, no chemical name | |
| PEPPER OIL BLACK SFO | Natural oil, no chemical name | |
| MINT OIL CRUDE ARVENSIS | Natural oil, no chemical name | |
| ROSE OIL TURKISH LOW METH EUGENOL LMR | Natural oil, no chemical name | |
| ROSE ABS TURK LOW METH EUG LMR | Natural oil, no chemical name | |
| ROSE OIL ISPARTA LOW ME LMR FOR LIFE | Natural oil, no chemical name | |
| ROSE CENTIFOLIA ABS MAROC LMR | Natural oil, no chemical name | |
| ROSE ESSENTIAL LMR XBOX FOR LIFE | Natural oil, no chemical name | |
| ROSE ESSENTIAL LOW ME LMR FOR LIFE | Natural oil, no chemical name | |
| ROSE ABS MD LMR | Natural oil, no chemical name | |
| ROSE ABS MAROC LMR | Natural oil, no chemical name | |
| ROSE OIL TURKISH LMR | Natural oil, no chemical name | |
| ROSE OIL BULGARIAN LMR | Natural oil, no chemical name | |
| ROSE ABS TURKISH LMR | Natural oil, no chemical name | |
| ROSE ABS VAH DIJON BLO | Natural oil, no chemical name | |
| ROSE ABS DAMASCENA PURE BLO | Natural oil, no chemical name | |
| SINENSAL NATURAL 20 EX ORANGE | Natural oil, no chemical name | |
| SCHINUS MOLLE OIL LMR SFO | Natural oil, no chemical name | |
| SCHINUS MOLLE ABS MD LMR | Natural oil, no chemical name | |
| SCHINUS MOLLE CO2 EXTRACT | Natural oil, no chemical name | |
| SPEARMINT OIL PURE | Natural oil, no chemical name | |
| STYRAX ESS PYROGENEE SB | Natural oil, no chemical name | |
| TOBAC ABS BALKAN LOW NICOTINE | Natural oil, no chemical name | |
| TAGETTE OIL MD LMR | Natural oil, no chemical name | |
| TAGETTE OIL EGYPT LMR | Natural oil, no chemical name | |
| THYME OIL WHITE SPAIN BLO | Natural oil, no chemical name | |
| TAGETE OIL MADAGASCAR LMR | Natural oil, no chemical name | |
| TUBEROSE ABS INDIA LMR | Natural oil, no chemical name | |
| VIOLET LEAF ABS EGYPT LMR | Natural oil, no chemical name | |
| VANILLA BEAN ABS MADAG | Natural oil, no chemical name | |
| WINTERGREEN OIL | Natural oil, no chemical name | |

In addition to the fragrances listed in Tables 1-2, the fragrance component may include 1, 2, 3, 4, 5, 6, 7, 8, 9 or more additional fragrance ingredients, if not already provided in Tables 1-2. Such additional fragrance ingredients include those described in US 2018/0325786 A1.

The additional fragrances, when combined with one or more fragrances of Table 1-2, constitute the fragrance composition. In this respect, the balance of the 100% by weight of the fragrance component is made up of one or more fragrances of Table 1-2 and optionally one or more additional fragrances.

When including one or a combination of the fragrances listed in Tables 1-2, at the specified amounts, the fragrance composition can be used in a consumer product at a significantly reduced dosage (*e.g.,* at 5- to 10-fold lower levels) as compared to a fragrance composition that does not include a fragrance listed in Tables 1-2, at the specified amount(s). In particular, the fragrance composition of this invention can be used at a dosage level of less than or equal to 1% of the total weight of a consumer product without significantly impacting fragrance performance, *i.e.,* perceived fragrance intensity, when compared to a fragrance composition that does not include a fragrance listed in Tables 1-2, at the specified amount(s). In some aspects, the fragrance composition is used at a dosage level of less than or equal 1%, 0.99%, 0.95%, 0.9%, 0.8%, 0.7%, 0.6%, 0.5%, 0.4%, 0.3%, 0.2%, 0.1% or 0.05% of the total weight of a consumer product, or any range delimited by any pair of the foregoing values.

The fragrance composition of this invention is of particular use in consumer products such as personal care products, fabric care products, or home fragrance products. When included in a consumer product, the fragrance composition of this invention improves one or more aesthetic features of the consumer product when compared to the same consumer product that includes a conventional fragrance composition, *i.e.,* a fragrance composition that does not include a fragrance listed in Tables 1-2, at the specified amount(s). Such aesthetic features include clarity, viscosity, color, flowability, and the like.

In some aspects, the consumer product is a personal care product. Examples of personal care products include, but are not limited to, shampoos, hair conditioners, personal washes such as soaps, body washes, personal cleaners and sanitizers. Personal care products can include, as active ingredients, one or more of a detersive surfactant, anti-dandruff agent, antimicrobial active, coloring agent or dye, hair bleaching agent, pharmaceutical active, hair growth or restorer agent, or hair conditioning agent.

Detersive surfactants provide cleaning performance to the composition. The detersive surfactant in turn comprises anionic detersive surfactant, zwitterionic or amphoteric detersive surfactant, or combinations thereof. Various examples and descriptions of detersive surfactants are set forth in US 2016/0228338. Examples include sodium lauryl ether sulfate, sodium lauryl sulfate, and ammonium lauryl sulfate. The concentration of the surfactant component in the personal care product should be sufficient to provide the desired cleaning and lather performance, and generally ranges from 0.5% to 50% (*e.g.*, 1% to 30%, 10% to 30%, 10% to 25%, 10% to 20%, 1% to 15%, and 12% to 22%). In particular embodiments, the consumer product has a low level of surfactant. As an illustration, the surfactant is present in a shower gel composition at a level of 10% to 20%, in a self-foaming personal wash product at a level of 1% to 15% by weight of the consumer product. When used in a liquid personal care product formulation, the fragrance composition of this invention can improve clarity and viscosity as compared to conventional fragrance compositions, an aesthetic feature which is maintained even upon storage for at least one month at elevated temperatures, *e.g.,* 45°C. In certain embodiments, the inclusion of a fragrance composition of this invention in a body wash provides for a level of clarity of less than 20 Nephelometric Turbidity Units (NTU). In particular embodiments, a level of clarity of less than 20 NTU is maintained for at least a month after storage at 45°C. In other embodiments, the inclusion of a fragrance composition of this invention in a body wash provides for a viscosity over a narrow range, *i.e.,* in the range of 10000 and 12000 mPas. In particular embodiments, the viscosity is maintained for at least a month after storage at 45°C.

In other aspects, the consumer product is a fabric care product. Examples of fabric care products include, but are not limited to, scent boosters, liquid or solid detergents, fabric conditioners, rinse conditioners, fabric liquid conditioners, tumble drier sheets, fabric refreshers, fabric refresher sprays, ironing liquids, and fabric softener systems. Scent boosters include those described in US 2007/0269651 Al and US 2014/0107010 Al. Fabric Care Products such as rinse conditioners, fabric liquid conditioners, tumble drier sheets, fabric refreshers, fabric refresher sprays, ironing liquids, and fabric softener systems are described in US 6,335,315, US 5,674,832, US 5,759,990, US 5,877,145, US 5,574,179, US 5,562,849, US 5,545,350, US 5,545,340, US 5,411,671, US 5,403,499, US 5,288,417, US 4,767,547 and US 4,424,134. Fabric care products may include, as fabric care active, a surfactant, bleach, enzyme, chelator, brightener, fabric softening agent and the like.

In some embodiments where the consumer product is a scent booster or liquid detergent, the inclusion of a fragrance composition of this invention significantly reduces discoloration, which is conventionally observed with a fragrance composition that does not include a fragrance listed in Tables 1-2, at the specified amount(s). In other embodiments where the consumer product is a powder detergent, the inclusion of a fragrance composition of this invention significantly reduces caking, as evidenced by a decrease in the presence of granules of greater than 1 mm in size. In further embodiments where the consumer product is a fabric conditioner, the level of consumer product active can be used in the range of between 1% and 20% by weight of the consumer product without significantly impacting performance.

In further aspects, the consumer product is a home fragrance product. Examples of home fragrance products include, but are not limited to, wax candles, gel candles and air fresheners. Home fragrance products may include, as active, a wax, gel, solvent, and the like. In embodiments where the consumer product is a wax candle, the candle exhibits reduced soot and volatile organic compound production compared to a candle including a fragrance composition that does not include a fragrance listed in Tables 1-2, at the specified amount(s).

The invention also provides methods for improving an aesthetic characteristic of a scented consumer product (*e.g.,* clarity, viscosity, color, etc.) by including in the consumer product a fragrance composition of this invention at a level of less than or equal to 1% by weight of the consumer product. Advantageously, the fragrance composition of this invention exhibits a perceived fragrance intensity that is parity with the fragrance intensity of a control composition (*i.e.,* a fragrance composition that does not include a fragrance listed in Tables 1-2, at the specified amount(s)) used at a level that that is 5- to 10-fold higher than the instant fragrance composition (*e.g.,* a level of at least 5% to 10% by weight of the consumer product). For the purposes of this invention, "perceived intensity," "perceived fragrance intensity," "perceived fragrance performance" or "perceived performance" are used interchangeably to refer to the intensity of a fragrance as perceived by a consumer. Such odor characteristics of a fragrance composition are typically assessed under different conditions by trained panelists that are capable of differentiating unambiguously the odor of a given fragrance composition under a first condition, for example during or after dilution of a perfumed product containing said fragrance composition, or on a substrate wetted with said product, from that of the same perfumed product, but under a second condition, for example after said product has dried on the substrate. Under such conditions, the difference is deemed to be consumer noticeable, that is, a majority of consumers will perceive the change of odor from said first condition to said second condition.

The following non-limiting examples are provided to further illustrate the present invention.

### Example 1: Product Transparency Improvement of Low Surfactant Formulations

*Product Aspect Assessment.* Regular fragrances were modified to include one or more high performing fragrance ingredients classified as high-performance ingredients. The ultra-high performing/impact modification versions were referred to as "High-Performance" (Table 3).

**TABLE 3**

| Regular Fragrance | Corresponding High-Performance Version |
|---|---|
| Great Gatsby | Gatsby High-Performance |
| Big Bird | Big Bird High-Performance |
| Flora Bella | Bella High-Performance |
| Happy Luxury | Amelia High-Performance |

The percentage of high-performance ingredients in each fragrance formulation is provided in Table 4.

**TABLE 4**

| Fragrance Formulation | | | High-performance Criteria | |
|---|---|---|---|---|
| | | | HI % (>5%) | High-performance% (>60%) |
| Great Gatsby | | | 1.31 | 17.17 |
| Gatsby High-Performance | | | 6 | 48.52 |
| Happy Luxury | | | 0.25 | 1.28 |
| Amelia High-Performance | | | 22.7 | 39.2 |
| Flora Bella | | | 0.06 | 2.81 |
| Bella High-Performance | | | 10.87 | 56.87 |
| Big Bird | | | 0.3 | 22.23 |
| Big Bird High-Performance | | | 1.2 | 58.4 |

Regular fragrances were applied at 1% and High-Performance fragrances were applied at 0.2% in a self-foaming base containing a low level of surfactant. Product clarity was compared for each pair of samples by visual inspection. In particular, the clarity of letters of a document placed behind the samples was measured. This analysis indicated that document letters were crisp and legible when read through a High-Performance fragrance formulation. By comparison, self-foaming products containing the regular fragrance appeared to be turbid right after the fragrance application such that letters behind each of the regular fragrance formulations appeared milky. Notably, the product turbidity appeared to worsen after 1 month at 45°C. However, the self-foaming products containing the High-Performance fragrances maintained product clarity even after 1 month storage at 45°C.

*UV-Vis Measurement.* The transmission of the samples was also measured using a spectrophotometer (Agilent CARY 8454 UV-Vis). Samples were pipetted into standard optical quartz UV-Vis cells with a path length of 10.0 mm. Loaded cells were placed in the spectrometer and the light transmission percentage was determined at 400 nm. The measured transmission percentage for each sample was converted to Nephelometric Turbidity Units (NTU) using the following equation: $NTU = 2.63 + 902.4 * \left(2-log\left(T%\right)\right) .$

See, Goodner (2009) *Estimating Turbidity (NTU) From Absorption Data*, Sensus Technical Note (SENTN-0010). Samples that were less than 20 NTU were considered to be transparent. Beyond that, samples were considered to be turbid. See US 5,662,893 A. The results of this analysis are presented in Table 5.

**TABLE 5**

| Formulation | Fresh Application Samples | Calculated NTU |
|---|---|---|
| Regular Versions | 1% Great Gatsby | 2279 |
| | 1% Big Bird | 409 |
| | 1% Flora Bella | 2279 |
| | 1% Happy Luxury | 1649 |
| Ultra Versions | 0.2% Gatsby High-Performance | 11 |
| | 0.2% Big Bird High-Performance | 7 |
| | 0.2% Bella High-Performance | 11 |
| | 0.2% Amelia High-Performance | 7 |

For freshly prepared application samples, all products with regular fragrance versions were more than 20 NTU and appeared turbid (assessed by three individuals). However, the turbidity of all Ultra versions was below 20 NTU and samples appeared to be transparent.

Samples were all placed in storage for up to 12 weeks at either 4°C, room temperature (RT) or 45°C. Product turbidity was subsequently assessed (Table 6).

**TABLE 6**

| Formulation | Calculated NTU | | |
|---|---|---|---|
| | 12 weeks at 4°C | 12 weeks at RT | 12 weeks at 45°C |
| Great Gatsby | 2167 | 2279 | 2167 |
| Big Bird | 403 | 462 | 374 |
| Flora Bella | 2306 | 2279 | 2279 |
| Happy Luxury | 1623 | 1649 | 1623 |
| Gatsby Ultra | 3 | 7 | 3 |
| Big Bird Ultra | 3 | 7 | 7 |
| Bella Ultra | 11 | 7 | 11 |
| Amelia Ultra | 3 | 3 | 3 |

All freshly prepared products with regular fragrances were more than 20 NTU and had turbid appearance. After 12 weeks of storage at 4°C, RT or 45°C, samples remained turbid. Comparison, High-Performance version formulations had a clear aspect at the start of the experiment and maintained product clarity after 12 weeks of storage at 4°C, RT and 45°C. These results clearly demonstrated the benefit of using high performing/impact creations as compared to their corresponding regular versions in a low surfactant level, self-foaming base.

*Product Fragrance Performance Evaluation***.** A performance evaluation was conducted with an expert panel (6 to 8 panelists). The following self-foaming samples (Table 7) were evaluated across four stages, namely lather, dry, point of purchase (POP) and cubicle bloom.

**TABLE 7**

| Regular Sample | High-Performance Version |
|---|---|
| 1% Great Gatsby | 0.2% Gatsby High-Performance |
| 1% Big Bird | 0.2% Big Bird High-Performance |
| 1% Flora Bella | 0.2% Bella High-Performance |
| 1% Happy Luxury | 0.2% Amelia High-Performance |

Evaluations were carried out with coded/blinded samples and comparisons were made for each pair of regular and High-Performance samples.

*Lather, Dry Evaluation Protocol.* To the left forearm of the wearer was applied 0.5 mL of the regular fragrance. The wearer then proceeded to lather his/her forearm area for 30 seconds. The same procedure was repeated for the High-Performance sample on the right forearm. Lather on both forearms were assessed by the expert panel for: Strength (scale from 0 to 10, 0 being odorless and 10 being extremely strong). Subsequently, the lather was rinsed off for 15 seconds under running water. Clean cotton towels were used to dry the forearms.

*POP (Point-of-Purchase) Evaluation Protocol*. Samples were blinded and expert panelists were asked to assess the POP stage by smelling from the product bottle directly.

*Cubicle Bloom Protocol.* Ten grams of sample were measured into a plastic bucket. A shower head was placed over the plastic bucket and the shower was turned on for 3 minutes. The shower was then turned off and cubicle bloom was assessed from a small window after 1 minute.

*Results*. Except for the Big Bird fragrance at bloom stage, all of the High-Performance fragrances performed at parity and even slightly more superior than the corresponding regular fragrances across evaluation stages (Table 8).

**TABLE 8**

| Formulation | Average Fragrance Intensity (±SD) | | | |
|---|---|---|---|---|
| | Bloom | Dry | Lather | POP |
| 1% Great Gatsby | 8.0±0.7 | 7.4±0.7 | 8.0±0.8 | 8.0±1.0 |
| 0.2% Gatsby High-Performance | 7.9±1.1 | 7.3±0.9 | 7.8±0.8 | 7.7±0.9 |
| 1% Big Bird | 8.0±0.1 | 6.7±0.7 | 7.5±0.8 | 8.1±0.7 |
| 0.2% Big Bird High-Performance | 6.9±0.6 | 6.7±0.6 | 7.7±0.7 | 7.7±0.8 |
| 1% Flora Bella | 7.5±0.4 | 7.1±0.5 | 7.5±0.8 | 7.7±0.5 |
| 0.2% Bella High-Performance | 8.2±0.6 | 7.5±1.2 | 8.2±0.9 | 7.9±1.5 |
| 1% Happy Luxury | 6.9±0.5 | 7.1±1.3 | 7.6±0.6 | 7.4±1.1 |
| 0.2% Amelia High-Performance | 8.4±0.6 | 7.6±1.0 | 8.4±0.6 | 8.5±0.6 |

Accordingly, despite being applied at a 5-times lower dose than the regular fragrance, the High-Performance versions were able to deliver parity or better performance. These results clearly demonstrated the effectiveness of High-Performance fragrances for product transparency improvement without compromising fragrance performance.

### Example 2: Product Viscosity Management for Liquid Personal Wash Applications

Fragrance addition can alter the viscosity of liquid bases by thickening or thinning. Viscosity and shear profile of liquid applications affect the flowability and ease of pouring of products. A comparative viscosity study was conducted using a shower gel base and four sets of regular and High-Performance fragrances applied at 1% and 0.2% respectively (Table 9). Product viscosity was measured using the rheometer (Anton Paar, MCR302). Stability tests were also conducted on these application samples at 45°C for up to a month.

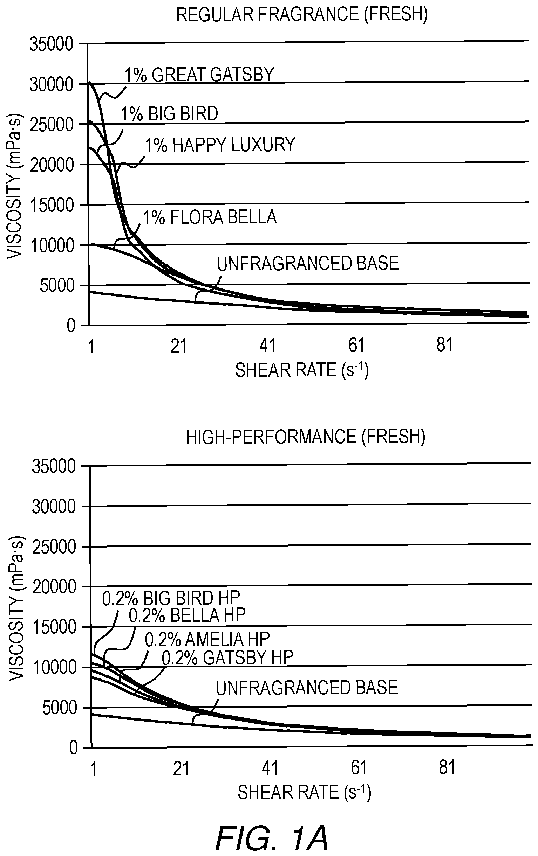

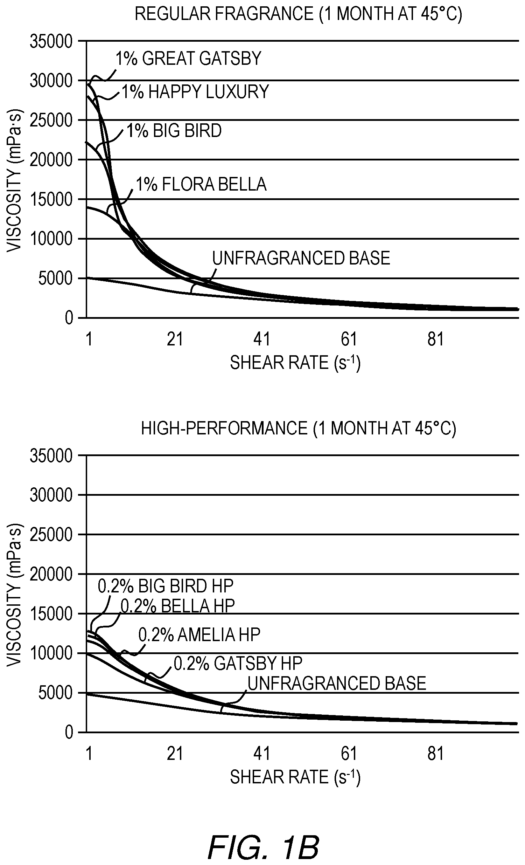

*Viscosity Measurement Results.* Based on the viscosity data presented in FIG. 1A, the addition of regular fragrances resulted in the fluctuation of product viscosity in the range of between 10000 and 30000 mPas. Products with regular fragrances were visibly more viscous and less easy to pour. However, the use of High-Performance fragrances minimized the viscosity deviation to a narrower range of between 10000 and 12000 mPas. The viscosity of the Ultra samples also was maintained in a stable and narrow range after a month at 45°C (FIG. 1B).

Product *Fragrance Performance Evaluation Protocol.* A performance evaluation was conducted with an expert panel (6 to 8 panelists). Shower gel samples containing 1% regular and 0.2% Ultra fragrances (Table 9) were evaluated across four stages (lather, dry, POP and cubicle bloom) using the same protocol and evaluation scale described in Example 1. Evaluations were carried out with coded/blinded samples. The sample containing the regular fragrance was compared to its corresponding Ultra sample.

*Evaluation Results*. All the High-Performance fragrances performed at parity or even slightly more superior than the corresponding regular fragrances across evaluation stages (Table 9).

**TABLE 9**

| Formulation | Average Fragrance Intensity (±SD) | | | |
|---|---|---|---|---|
| | Bloom | Dry | Lather | POP |
| 1% Great Gatsby | 7.1±1.0 | 6.8±0.7 | 7.6±0.8 | 7.4±0.8 |
| 0.2% Gatsby High-Performance | 7.0±1.2 | 6.5±0.6 | 7.4±0.7 | 7.1±0.8 |
| 1% Big Bird | 7.0±0.8 | 6.4±0.9 | 7.6±0.5 | 7.7±0.6 |
| 0.2% Big Bird High-Performance | 6.1±1.0 | 6.0±0.7 | 7.1±0.9 | 6.9±0.6 |
| 1% Flora Bella | 6.1±1.0 | 6.8±0.9 | 7.2±0.7 | 7.1±0.5 |
| 0.2% Bella High-Performance | 7.3±0.9 | 6.6±0.9 | 7.7±0.9 | 7.6±1.2 |
| 1% Happy Luxury | 6.4±1.1 | 6.3±0.7 | 7.3±0.4 | 7.2±0.7 |
| 0.2% Amelia High-Performance | 7.7±0.9 | 7.1±0.6 | 7.9±0.7 | 7.9±0.6 |

The usage of the High-Performance fragrances enabled product viscosity management over a narrower fluctuation range and High-Performance fragrances performed at parity to the regular fragrances across all evaluation stages. Thus, additional technical benefit was delivered without compromising performance.

### Example 3: Reduced Scent Booster Product Discoloration with High-Performance Fragrances

The objective of this study was to determine whether a High-Performance fragrance neat oil dosed at 40% (10 g) was at parity in fragrance strength with a traditional scent dosed at 100% (25 g) in damp and heat-dry stages.

*Product Preparation.* Scent booster samples were prepared by adding a traditional neat oil (Benchmark Fragrance 2) or a High-Performance fragrance neat oil into warmed polyethylene glycol (PEG) 8000 as a base (Table 10).

**TABLE 10**

| Sample | Neat Fragrance Oil | PEG 8000 |
|---|---|---|
| 1 | 0.9% High-Performance Oil | 99.1% |
| 2 | 9.0% Traditional Oil | 91.0% |

While the batch was still hot, pastilles were created on a clean, flat, stainless steel surface with a 1 mL syringe. A four-pound load of laundry composed of 10 small towels (12" x 12", 86% cotton/14% polyester face) and one large towel (48" x 26", 86% cotton/14% polyester ballast) was loaded into a front loader washing machine along with the scent booster samples and detergent. The towels were washed in cold water on the permanent press cycle. Several damp towels were removed, folded and stored in closed trays for subsequent evaluation. The remaining laundry was dried in a clothes drier for 60 minutes under medium heat. Dry face towels were folded and stacked in an open tray for subsequent evaluation.

*Product Fragrance Performance Evaluation*. A performance evaluation was conducted with an expert panel (5 to 10 panelists). The samples were evaluated across two stages, namely damp and dry. Fragrance intensity was rated on a scale of 0 to 5, wherein 0 = Smell Nothing and 5 = Extremely Strong. Two separate experiments were conducted and reported in Table 11.

*Results.* As seen in Table 11, the High-Performance fragrance at 10 g was parity to the traditional fragrance at damp and heat-dry stages.

**TABLE 11**

| Fragrance | Fragrance Intensity | | | |
|---|---|---|---|---|
| | Damp | | Heat-Dry | |
| Traditional | 3.67 | 4.07 | 2.42 | 3.35 |
| High-Performance | 4.08 | 4.42 | 3.08 | 3.87 |

These results demonstrated that the use of 60% less scent booster product with a High-Performance fragrance yielded the same fragrance intensity as 100% of a traditional fragrance.

Fragrance performance was also determined after the samples had been aged at 4°C and 37°C for about 2 weeks. Two separate experiments were conducted and reported in Table 12. The results of this analysis indicated that the High-Performance samples at 0.9% were parity in fragrance intensity to the traditional fragrance at 9.0%.

**TABLE 12**

| Fragrance | Fragrance Intensity | | | |
|---|---|---|---|---|
| | Damp | | Dry | |
| | 4°C | 37°C | 4°C | 37°C |
| Traditional | 3.40 | 3.44 | 2.77 | 2.57 |
| | 2.84 | 2.70 | 2.14 | 2.13 |
| High-Performance | 4.24 | 4.09 | 3.56 | 3.51 |
| | 3.13 | 3.09 | 2.53 | 2.49 |

These results demonstrated that the use of 10% High-Performance fragrance can yield the same fragrance intensity as 100% of a traditional fragrance even with aged samples.

To assess whether there were any physical differences in samples prepared with traditional and High-Performance fragrances, scent booster pastilles were prepared as described above with 0.9% and 9.0% fragrance loads. All samples were then aged at 4°C and 37°C for about 2 weeks.

The results of this analysis (Table 13) indicated that scent booster samples composed of 0.9% fragrance load had a higher melting point compared to the samples at 9.0%. Thus, a lower dosage increased the physical stability of the samples. In addition, it was noted that all samples dosed at 0.9% had less discoloration than samples dosed at 9.0%.

**TABLE 13**

| Fragrance | Dose | Melting Point after Storage at: | |
|---|---|---|---|
| | | 4°C | 37°C |
| Traditional | 0.9% | 63.36°C | 63.13°C |
| | 9.0% | 59.42°C | 60.10°C |
| High-Performance | 0.9% | 62.32°C | 61.66°C |
| | 9.0% | 59.83°C | 59.89°C |

### Example 4: Reduced Liquid Detergent Discoloration with High-Performance Fragrances

The objective of this study was to observe any differences in performance (fragrance intensity) in unit dose detergent samples at 0.2% and 2.0% that have been aged at 4°C and 37°C for about 3 weeks.

*Product Preparation*. Detergent samples were prepared by adding a traditional neat oil (Benchmark Fragrance 2) or a High-Performance fragrance neat oil into an unfragranced liquid base (Table 14). Fragrance performance was determined after the samples had been aged at 4°C and 37°C for about 3 weeks.

**TABLE 14**

| Sample | Neat Fragrance Oil | Liquid Base |
|---|---|---|
| 1 | 0.2% High-Performance Oil | 99.8% |
| 2 | 2.0% Benchmark Fragrance 2 | 98.0% |

Towels were washed with the liquid detergent in accordance with the method described in Example 3 to obtain towels at both damp and dry stages. The results of this analysis (Table 15) indicated that samples aged at the higher temperature, 37°C, performed at parity to the samples aged at 4°C. In addition, High-Performance samples at 0.2% were parity in fragrance intensity to the traditional fragrance at 2.0%. Moreover, all samples dosed at 0.2% had less discoloration than samples dosed at 2.0%.

**TABLE 15**

| Fragrance | Fragrance Intensity | | | |
|---|---|---|---|---|
| | Damp | | Dry | |
| | 4°C | 37°C | 4°C | 37°C |
| 2% Traditional | 3.09 | 2.87 | 2.27 | 1.93 |
| 2% High-Performance | 3.53 | 3.10 | 3.06 | 2.79 |
| 0.2% Traditional | 2.46 | 2.16 | 1.86 | 1.77 |
| 0.2% High-Performance | 2.67 | 3.07 | 2.33 | 2.31 |

Thus, use of 10% High-Performance fragrance can yield the same fragrance intensity as 100% of a traditional fragrance even with aged samples.

### Example 5: Reduced Powdered Detergent Caking with High-Performance Fragrances

With the design of High-Performance fragrances, the performance can match closely to a standard fragrance with one tenth of the standard dosage. By lowering the fragrance dosage, the requirement of powder detergent base to uphold the fragrance oil is therefore lowered, and the possibility of sticky/caking powder is lowered. At the same time, the performance is not affected due to the strong performance from High-Performance fragrance. Hence, customers will have less issue about the base odor coverage and olfactive performance.

To demonstrate the anti-caking feature of the High-Performance fragrances, four selected fragrance oils (two High-Performance and two regular scents; Tables 16 and 17) were dosed manually into an unfragranced detergent powder base at defined dosage levels (Table 18).

**TABLE 16**

| Ingredient | Cavalier High-Performance | Cavalier (Regular) |
|---|---|---|
| ACETATE C-06 | 1 | 1 |
| ALD AA TRIPLAL BHT | 20 | 7 |
| ALD C-10 TOCO | 100 | 4.7 |
| ALD C-11 UNDECYLIC TOCO | 50 | 17 |
| ALD C-12 LAURIC TOCO | 75 | 5.5 |
| ALD C-12 MNA TOCO | 50 | 6 |
| ALD C-8 TOCO | 75 | 3.5 |
| AMBER CORE (ELINCS) BHT | 1 | 1.5 |
| AMBER XTREME TM | 2.5 | |
| AMBERMOR EX | 1 | 0.5 |
| AMBERTONIC | 5 | |
| AUBEPINE | 1 | 1 |
| AURAWOOD (ELINCS) 10% DPG | | 2.5 |
| BACDANOL TOCO | | 3 |
| CALONE 10% DPG | 1 | 2.5 |
| CAMPHOR PWD SYN | 7 | 2 |
| CITRATHAL 85 PCT ETOH BHT | | 1 |
| CITRONELLOL 950 | 10 | 17.5 |
| CP FORMATE APHERMATE | | 1 |
| CYCLACET | | 95 |
| CYCLAPROP | 75 | 30 |
| DAMASCONE DELTA BHT | 2.5 | 1 |
| DECENAL,CIS-4 1% DPG | | 5 |
| DECENAL,CIS-4 | 1 | |
| DIHYDRO MYRCENOL | 35 | 98.24 |
| DIPHEN OXIDE | 15 | 1 |
| DIPROPYLENE GLYCOL | | 127.56 |
| DOREMOX | | 0.5 |
| EBANOL BHT | | 1 |
| ETHYLENE BRASSYLATE(ASTRATONE) | | 70 |
| EUCALYPTOL USP | 55 | 1 |
| FRUITATE (ELINCS) | | 1 |
| FLORAL SUPER | 1 | |
| GERANIOL 980 PURE | 4.5 | 9 |
| HEXYL SAL | | 3 |
| HEXYL CINN ALD TBHQ | | 150 |
| IONONE BETA EXTRA | 1 | |
| ISO AMYL ACET | 1 | 0.5 |
| ISO GAMMA SUPER TOCO | | 5.5 |
| ISO PROPYL-2-METH BUTY | 5 | 2 |
| Javamor TT (ELINCS) | 1 | |
| LIMONENE D TOCO | 12.5 | 40 |
| LINALOOL SYN TOCO | 30 | 90 |
| LILIAL TOCO | | 55 |
| METH ANTH BHT (USDEA) | 1 | |
| METH ANTH BHT (USDEA) 10% DPG | | 5 |
| METH BENZOATE | 5 | 1 |
| METH BETA NAPH KETONE | 2.5 | 3 |
| METH CEDRYL KETONE | 5 | 20 |
| METH DH JASMONATE | | 4 |
| METH IONONE GAMMA A TOCO | 2 | |
| METH OCTIN CARBONATE 1% DPG | 1 | 2.5 |
| MINT OIL CRUDE ARVENSIS | 14 | 0.5 |
| MUSKALACTONE | 1 | |
| MUSKALACTONE REPL FC, BS, HC (W/O SINF) | | 3 |
| NEROL 700 | 3.5 | |
| NEROL BG REPL MATCH 142460 | | 7 |
| ORANGE OIL CP TOCO | 7.5 | 22.5 |
| PATCHONE | 1 | 2.5 |
| PATCHOULI OIL LIGHT BLO | 3 | 1 |
| PHEN ETH ALC WHITE EXTRA (MXDEA) | 14 | 55 |
| SINO CITRYL MATCH 00194215 | 1 | |
| SINO CITRYL 10% DPG | | 5 |
| TERPINEOL ALPHA JAX | 4.5 | 18 |
| TERPINYL ACET JAX | 37.5 | 115 |
| TETRAHYDRO LINALOOL | | 10 |
| VERDOX | 7.5 | 25 |
| VERTENEX | | 25 |
| YARA YARA | 50 | 11.5 |
| Total | 800 | 1200 |

**TABLE 17**

| Ingredient | High-performance 2 | HKRND (Regular) |
|---|---|---|
| TRISAMBER (ELINCS) | 250 | |
| ALD C-18 | 100 | |
| JAVANOL TT (ELINCS) | 80 | |
| UNDECALACTONE,DELTA | 66 | |
| HEXADECANOLIDE | 50 | |
| UNDECAVERTOL TOCO | 33 | 13 |
| PINO ACETALD TOCO | 30 | |
| MUSCENONE (ELINCS) | 20 | |
| AMBER XTREME TM | 16.5 | |
| OCEANOL | 15 | |
| METH BENZOATE | 14 | 3.4 |
| AMBERMOR EX | 10 | |
| BERGAMAL TOCO | 10 | |
| VIOLIFF BHT (ELINCS) | 10 | 1.9 |
| VELTOL PLUS | 10 | |
| GUAIACOL LIQ SD 1% DPG | 7.5 | |
| SINENSAL NATURAL 20 EX ORANGE | 7 | |
| STYRALYL ACET | 5.5 | 8.3 |
| ISO BUTYL QUINOLINE 10% DPG | 3.5 | |
| DELPHONE | 2 | 2.8 |
| CYCLACET | | 127 |
| VERDOX | | 83 |
| DIHYDRO MYRCENOL | | 63.5 |
| VERTENEX | | 62.5 |
| CYCLAPROP | | 56 |
| ISO BORNYL ACET | | 55 |
| ISO E SUPER TOCO | | 48.5 |
| YARA YARA | | 38 |
| IONONE BETA EXTRA | | 36.5 |
| ORANGE OIL CP TOCO | | 33 |
| TETRAHYDRO LINALOOL | | 26 |
| TETRAHYDRO MYRCENOL | | 26 |
| DIPHEN OXIDE | | 24.5 |
| BACDANOL TOCO | | 24 |
| ALD AA TRIPLAL BHT | | 21.5 |
| BENZ ACET | | 18 |
| EUCALYPTOL USP | | 17.5 |
| TERPINEOL ALPHA JAX | | 17 |
| ALD C-12 MNA TOCO | | 15 |
| ALLYL HEPTANOATE | | 14 |
| ETH-2-METH BUTY | | 11.5 |
| AGRUNITRILE | | 11.5 |
| FRUCTALATE (ELINCS) | | 9.5 |
| BENZ ACETONE | | 8.8 |
| FLORIFFOL (ELINCS) TOCO | | 7.6 |
| ACETATE C-06 | | 7.2 |
| ALD C-10 TOCO78 | | 6.2 |
| CITRAL REFINED HLR | | 5.8 |
| DIMETH BENZ CARB ACET | | 5.6 |
| ALD C-12 LAURIC TOCO | | 5.3 |
| MANZANATE | | 5.3 |
| CITRONELLOL 950 | | 4.5 |
| AUBEPINE | | 4.4 |
| AMYL SAL | | 3.8 |
| METH ACETOPHENONE | | 3.5 |
| CYCLAMAL TOCO | | 3.3 |
| ALD C-11 INTRELEVEN (TT) PRG | | 3.1 |
| METH ANTH BHT (USDEA) | | 2.8 |
| BENZ ALC | | 2.8 |
| DAMASCONE DELTA BHT | | 2.6 |
| ALD C-11 UNDECYLIC TOCO | | 2.2 |
| LIME OIL WI TYPE TOCO | | 2 |
| CAMPHOR PWD SYN | | 1.6 |
| CETALOR | | 1.6 |
| IONONE EPOXIDE,BETA | | 1.4 |
| METH PHEN ETH ETHER | | 1.2 |
| METH DH JASMONATE | | 1.2 |
| MELAFLEUR BHT | | 1.2 |
| ISO PROPYL MYRISTATE | | 1.2 |
| METH PARA CRESOL | | 1.2 |
| GALBASCONE ALPHA 95 PRG | | 1.1 |
| CYCLOHEXYL SAL (ELINCS) | | 1 |
| LINALYL ACET | | 0.7 |
| JASMONE CIS RB LRG-1219 | | 0.7 |
| PHEN ETH ACET | | 0.4 |

**TABLE 18**

| Fragrance | Dosage | % High-Performance | | | |
|---|---|---|---|---|---|
| | | | | | |
| Cavalier High-Performance | 0.06% | 75.14 | | | |
| Cavalier | 0.60% | 7.56 | | | |
| High-Performance 2 | 0.06% | 61.3 | | | |
| HKRND | 0.60 | 25.43 | | | |

An exemplary powder detergent formulation is provided in Table 19.

**TABLE 19**

| Ingredient | Weight (%) |
|---|---|
| Sodium Carbonate | 81.9 |
| Ethoxylated C12-C15 alcohol sulfate salt | 4.3 |
| C12-C15 alcohol ethoxylate | 2.4 |
| Sodium Sulfate | 1.5 |
| Sodium bicarbonate | 1.3 |
| Sodium polyacrylate | 0.7 |
| Sodium Carboxymethylcellulose | 0.1 |
| Optical Brightener | 0.2 |
| Perfume | 0.1 |
| Polyvinyl Alcohol | 0.1 |
| Water | 7.4 |

| | |
|---|---|
| Taken from US 5,443,751. | |

Samples were stored in 4°C and 45°C for 12 weeks. After 12 weeks, samples were conditioned to ambient temperature then sieved using a 1 mm size sieve. Large granules, which are indicative of sticky powder, do not pass through such a sieve. The weight of the large granules was calculated according to the total weight of sample. This percentage was used as an indication of the level of caking. The results of this analysis are presented in Table 20.

**TABLE 20**

| Storage Conditions | Sample | < 1 mm (g) | > 1 mm (g) | Caked (%) |
|---|---|---|---|---|
| 45°C for 12 weeks | Cavalier Ultra | 19.25 | 0.73 | 3.65 |
| | Cavalier | 17.83 | 2.11 | 10.58 |
| | High-Performance 2 | 19.84 | 0.62 | 3.03 |
| | HKRND | 18.34 | 1.64 | 8.21 |
| 4°C for 12 weeks | Cavalier Ultra | 19.38 | 0.66 | 3.29 |
| | Cavalier | 18.08 | 1.98 | 9.87 |
| | High-Performance 2 | 19.93 | 0.57 | 2.78 |
| | HKRND | 18.72 | 1.95 | 9.43 |

This analysis indicated that regular fragrances, Cavalier and HKRND, had more >1 mm powder than the samples including the High-Performance fragrances.

In addition to caking, sensory evaluations were conducted. Only the POP stage was evaluated by an expert panel for intensity differences. The results of this analysis are presented in Table 21.

**TABLE 21**

| Storage Conditions | Sample | Intensity |
|---|---|---|
| 45°C for 12 weeks | Cavalier Ultra | 2.52 |
| | Cavalier | 3.45 |
| | High-Performance 2 | 2.78 |
| | HKRND | 3.80 |
| 4°C for 12 weeks | Cavalier Ultra | 3.02 |
| | Cavalier | 3.48 |
| | High-Performance 2 | 3.47 |
| | HKRND | 3.43 |

Based on the above assessment of caking in powder detergent, it was concluded that using a low level of High-Performance fragrance reduces the amount of large powder granule formation, therefore lowering the possibility of powder caking compared to a standard fragrance. The High-Performance fragrance is able to lower the level of fragrance to one tenth of the standard fragrance, hence bringing the additional benefit of lower caking tendency in powder.

### Example 6: Visco-Stability Improvement with Encapsulated High-Performance Fragrances

The addition of capsules into a fabric conditioner base causes disruptions to the system which can affect the technical parameters of the system (*e.g.,* viscosity, etc.). Therefore, it was determined what effect the use of an encapsulated High-Performance fragrance would have on visco-stability of a consumer product.

Samples of slurry, water and base were made for measuring visco-stability over time. The capsules used were all melamine formaldehyde capsules. Three regular fragrances were used. Products were tested in a 19% active level fabric conditioner base (Table 22) as is, and this was also diluted with water to create a 12% active level base.

**TABLE 22**

| Material | Amount (g) |
|---|---|
| REWOQUAT^{®} WE 18 (Esterquat; Evonik, 90% in IPA) | 23.46 |
| PROXEL^{®} GLX (1,2-benzisothiazolin-3-one) | 0.11 |
| Water | 76.1 |
| Calcium Chloride (25%) | 0.33 |

For the initial sensory analysis, samples were washed using a front loader washing machine. Wash loads of 2.2 kg, including big towels, T-shirts, pillow cases, dish towels, and evaluation towels (cotton 30cm x 30cm), were used. The laundry was washed at 40°C for 60 minutes using 15.5 liters of water, with two 17-liter rinses. The cloths were first washed with Persil non-bio detergent (70 g) and then with the fabric conditioner dosed at 36 g. Cloths were line dried.

Samples were evaluated using the trained sensory panel at dry pre and gentle handling stages. Samples were assessed blind and with replicates against a benchmark (*i.e.,* a second Jillz sample). Evaluators rated the performance (strength) of the fragrance using an LMS scale. JMP statistical software was used for data analysis and two-way ANOVA, Fit Model is used.

For the visco-stability, samples were stored at four temperatures: 5°C, 20°C (room temperature), 37°C and 40°C. Viscosity was measured initially (t = 0 days) and then periodically at 4-week intervals. Viscosity was measured using an HTR 302 compact machine and measurements were taken at shear rates of 2/s, 20/s & 106/s (rotations per second). When using an encapsulated High-Performance fragrance, a lower dose of slurry can be used thereby minimizing interruptions to the total product formula while maintaining parity or increased performance. The results showed that, particularly in high active level bases, using a High-Performance capsule improves the visco-stability of the total product at elevated temperatures.

When introducing High-Performance capsules into a fabric conditioner base, overall product viscosity will not increase over time thereby reducing other downstream visual aspects of the product including flowability/pourability, color, odor intensity, etc.

### Example 7: Reduced Need for Deposition Aid with High-Performance Fragrances

In a fabric conditioner system, the quat (or "active") in the base acts not only as a softener, but also as a deposition aid for a neat oil fragrance introduced to the system. The level of quat differs between products, which can have a large effect on the amount of deposition aid available for a neat oil fragrance.

Four different active concentration bases were created, using the same 19% active level base described in Example 6. The 19% concentration was used, and then the base was diluted with water to 12%, 8% and 2% active levels.

Two High-Performance fragrances were used, High-Performance 2 (Table 17) and High-Performance 3 (Table 23), alongside two commercial benchmarks (Table 24). The commercial fragrances were dosed at 1.0%, whereas the High-Performance fragrances were dosed at 0.1%. Samples were washed and evaluated with a trained sensory panel.

**TABLE 23**

| Ingredient | Benchmark Fragrance 1 (ppt) | Benchmark fragrance 2(ppt) | High-Performance fragrance (ppt) | |
|---|---|---|---|---|
| | | | 3 | 2 |
| Acetate C-06 | | 40 | | |
| Acetophenone | | 4 | | |
| Adoxal TOCO | | 5.5 | | |
| Ald AA Triplal BHT | | 9.2 | | |
| Ald C-11 Ulenic TOCO | 5.5 | 28 | | |
| Ald C-12 Lauric TOCO | 7.5 | 38.5 | | |
| Ald C-12 MNA TOCO | 6.2 | 30.5 | 50 | |
| Ald C-18 | | | | 100 |
| Allyl Phenoxy Acet | | 4.4 | | |
| Amber Core (ELINCS) BHT | 0.5 | | | |
| Amber Xtreme^{™} | 1.5 | | | 16.5 |
| Ambermor Ex | | 1.2 | 100 | 10 |
| Ambermor Ex | | | | |
| Amyl Sal | 13.8 | 23.5 | | |
| Anethole USP BHT | 1.1 | | | |
| Arbanol Glid | 2 | | | |
| Aubepine | | 12.5 | | |
| Bacdanol TOCO | 1.4 | | | |
| Benz Acet | 2.1 | 28.5 | | |
| Benz Acetone | | 1.6 | | |
| Benz Sal | 77 | 2.7 | | |
| Benzoic Acid | 0.3 | | | |
| Bergamal TOCO | | | | 10 |
| Borneol 10% DPG | 0.5 | | | |
| Butyl Acet | | 0.3 | | |
| Camphor PWD SYN | 1.9 | | | |
| Canthoxal Tocopherol | 0.2 | | | |
| Carvacrol CP 10% DPG | 1 | | | |
| Caryophyllene | 4.3 | | | |
| Cashmeran | | | 100 | |
| Cedarwood Type Light Texas | 1 | | | |
| Cedramber | | 0.2 | | |
| Cinn Alc | 0.6 | | | |
| Citral Refined HLR | 4 | | | |
| Citrathal 85 PCT ETOH BHT | 5 | | | |
| Citronellol 950 | | 14.5 | | |
| Citronellyl Acet | | 0.2 | | |
| Citronellyl Formate 10% IPM | 1 | | | |
| Coranol (ELINCS) | | 2.3 | | |
| Coumarin | 9.7 | | | |
| Cuminic Ald TOCO 10% DPG | 1 | | | |
| Cyclamal TOCO | 12.5 | 33 | | |
| Cyclaprop | | 0.5 | | |
| Cyclohexyl Sal (ELINCS) | | 2.5 | | |
| Damascenone Total TOCO | 0.2 | | | |
| Damascone Delta BHT | 2.6 | 3 | | |
| Decalactone Gamma | | 5.3 | | |
| Delphone | | | | 2 |
| Dihydro Myrcenol | | 128.5 | | |
| Dimeth Benz Carb Acet | | 26.5 | | |
| Dowanol DPM | 1.3 | 1.1 | | |
| Eth Linalool TOCO | | 0.3 | | |
| Eth Vanillin | 7.2 | 0.8 | | |
| Ethylene Brassylate (Astratone) | | 1.4 | | |
| Eugenol Nat EX Clove leaf Oil | 28.5 | 24 | | |
| Florhydral BHT TOCO (ELINCS) | | 3.6 | | |
| Floriffol (ELINCS) TOCO | 184 | 24.5 | | |
| Galbascone Alpha 95 PRG | | 0.6 | | |
| Galbascone PRG BHT | 0.7 | | | |
| Gauiacol Liq SD 1% DPG | | | | 7.5 |
| Geraniol 980 Pure | 3.1 | 10.5 | | |
| Geranyl Acet Pure | 15.3 | | | |
| Geranyl Prop | | 0.4 | | |
| Guaiacwood Oil | 1 | | | |
| Habanolide (ELINCS) | 23 | 10.5 | | |
| Helional | | 0.2 | | |
| Heliotropine (piperonal) (USDEA) | 1.3 | 3.2 | | |
| Helvetolide (ELINCS) | | 14.5 | | |
| Hexadecanolide | | | | 50 |
| Hexyl Cinn Ald TBHQ | 234 | 40 | | |
| Hexyl Sal | 41 | 19.5 | | |
| Hydratropic Alc Coeur | 0.5 | | | |
| Hydroxycit Pure Syn | 0.3 | | | |
| Indisan neat | 1.6 | | | |
| lonone Alpha BHT | | 8.1 | | |
| Iso Amyl Buty | | 5.7 | | |
| Iso Butyl Quinoline 10% CFLEX#2 | | 1 | | |
| Iso Butyl Quinoline 10% DPG | | | | 3.5 |
| Iso E Super TOCO | 67 | 9 | | |
| Iso Eugenol | 0.2 | | | |
| Javanol TT (ELINCS) | | | | 80 |
| Linalool Pure Ex Pinene | | 136 | | |
| Linalool Syn TOCO | 16.5 | | | |
| Linalyl Acet | | 0.5 | | |
| Lindenol | 0.5 | 4 | | |
| Meijiff | 0.6 | | | |
| Meth Anth BHT (USDEA) | 0.2 | | | |
| Meth Benzoate | 0.5 | | | 14 |
| Meth Beta Naph Ketone | 2.5 | | | |
| Meth Cedryl Ketone | | 0.4 | | |
| Meth DH Jasmonate | 16.6 | 31 | | |
| Meth lonone Gamma A TOCO | 77 | | | |
| Meth Nonyl Ketone | 0.2 | 0.6 | | |
| Meth Para Cresol | 0.4 | | | |
| Muscemor (ELINCS) | | | 100 | |
| Muscenone (ELINCS) | | | | 20 |
| Nerol Super Vernol | 1.3 | 5.8 | | |
| Neryl Acet A | 7.2 | | | |
| Oceanol | | | | 15 |
| Orange Terpenes Ex 5X TOCO | 15 | | | |
| Patchone | 10 | | | |
| Patchouli Oil Light BLO | 12.5 | 65 | | |
| Paxamber BHT | | 1 | | |
| Peonile (ELINCS) | 37.5 | 0.7 | | |
| Phen Eth Alc White Extra (MXDEA) | 3.3 | 22 | | |
| Phen Eth Sal | 3.2 | | | |
| Phenoxanol | | 7.3 | | |
| Phenoxy Ethyl Iso Butyrate | | 0.5 | | |
| Pino Acetald TOCO | | | 25 | 30 |
| Plinol Special | 0.3 | | | |
| Rose Oxide TOCO | 2 | 12.5 | | |
| Rosethyl | | | 100 | |
| Scentenal (ELINCS) BHT | 1 | | | |
| Sinensal Natural 20 Ex Orange | | | | 7 |
| Styralyl Acet | | 0.6 | | 5.5 |
| Terpinyl Acet Jax | 1 | | | |
| Trisamber (ELINCS) | | | | 250 |
| Undecalactone Delta | | | | 66 |
| Undecalactone Gamma Coeur | 6.5 | 0.9 | | |
| Undecavertol TOCO | | 37 | | 33 |
| Veltol Plus | | | | 10 |
| Veramoss | 0.5 | | | |
| Vertenex | | 47.5 | | |
| Vertoliff | 5 | | | |
| Violiff BHT (ELINCS) | | | | 10 |
| Yara Yara Extra PRG | | | 25 | |
| Ysamber K (ELINCS) | 0.7 | 0.7 | | |

Table 24 details the types of fragrances in the fragrance formulations.

**TABLE 24**

| Fragrance | High-performance % | Odor Potency |
|---|---|---|
| Benchmark Fragrance 1 | 8.93 | 531 |
| Benchmark Fragrance 2 | 29.71 | 1311 |
| High-Performance 3 | 100 | 13729 |
| High-Performance 2 | 61.33 | 13861 |

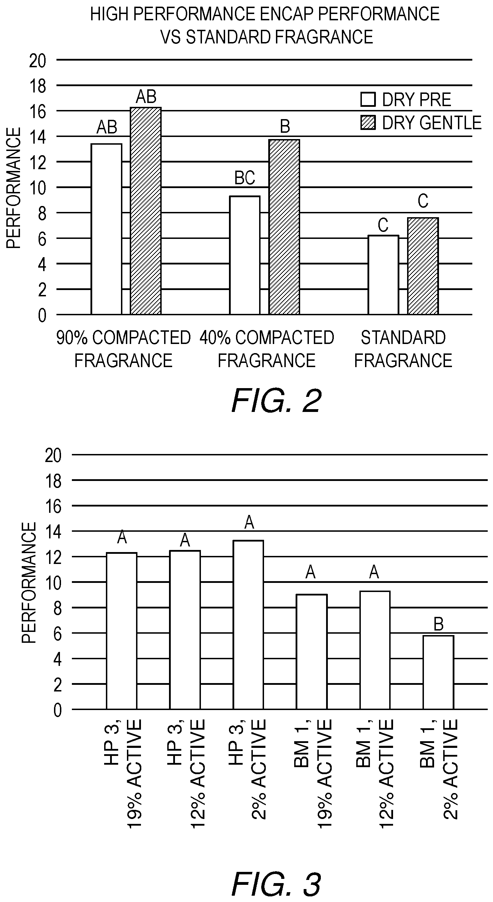

Sensory results of selected fragrances/active levels are presented in FIG. 3. These results indicate that High-Performance 3 does not lose performance at the highest and lowest active levels. By comparison, lowering the active level of Benchmark Fragrance 1 results in a statistically significant drop in performance compared to the higher active levels.

The data herein demonstrate that when using a High-Performance fragrance, deposition aid levels can be reduced. Therefore, parity or higher performance can be achieved in with a fabric conditioning active at a level of 2% to 19%.

### Example 8: High-Performance Fragrances in Candle Application

Burning candles, particularly scented candles, has always been known to produce unwanted soot, and recently the emissions in the form of volatile organic compounds (VOCs) have come under scrutiny as an undesirable effect from burning candles. The formation of soot and VOCs in candle emissions are a result from incomplete combustion of the melted wax and fragrance fuel in the flame. This invention circumvents these problems by having a lower dosage of fragrance in the candle which results in less emissions, but maintains the same or better level of fragrance strength and performance.

Having less fragrance oil in the candle, provides several benefits. The burn performance of the candle improves by the rate of consumption having more consistency across the life of the candle, mainly due to the flame staying at an optimal, consistent height during burn. There are also less undesirable wick effects such as mushrooming and smoldering. Several wicks usually need to be tested to optimize flame height in a standard candle due to the fragrance oil's impact of the wick effectiveness. However, when there is less fragrance, there is less concern about wick performance being affected by fragrance. Less fragrance dosage also circumvents oil weeping from the candle, the candle being too soft, and the potential for flash over.

To demonstrate the use of a High-Performance fragrance in a candle application, soot production was measured using the European Standard: Candles-Specifications for Sooting Behaviour EN 15426. The soot was collected by placing the candle on a lab stand in a mesh cylinder 32 cm high and a diameter of 25 cm with a 4x4 inch glass plate top, leaving 5 cm gap between the bottom of the cylinder and the bottom of the lab stand to allow for air flow. The candle was burned for 4 hours and the soot was collected on the plate. The plate was then placed in an enclosed wooden measuring chamber with a light source under the plate and a digital lux meter (Dr. Meter model LX1330B) on top measuring the illuminance going through the glass plate. The ratio of illuminance of sooted plate (E₃) vs. clean plate (E₁) is called the soot index (Sᵢ). The smaller the soot index, the less soot has been collected from the burning candle.

VOC emissions in the form of benzene and naphthalene were measured via headspace collection on duplicate Tenax sorbent tube with analysis by thermal desorption onto a GC/MS. The candle was placed in a 0.03 m³ acrylic chamber in a purged booth. The air flow/mixing fan was connected to a variac variable transformer set to 25-30 volts, creating a 0.3 m/s velocity. The candle was burned for 1 hour to ensure that the headspace became saturated in equilibrium. The headspace was collected onto the duplicate tubes via a battery hand-help pump at a flow of 200 mL/min for 20 minutes, a total volume of 4 L. The level of benzene and naphthalene found in each sample was determined by integrating the area count of each peak. To have more accuracy, only ion 78 was used to determine the benzene level and ion 128 was used to determine the naphthalene level. The area count on the GC/MS chromatograph was used to calculate the vapor concentration (in µg/m³) of each emission substance in the headspace. Duplicates were checked for reproducibility by determining the % Relative Standard Deviation.

The standard candle for the intensity evaluations was prepared by placing a wick (size 44-18-24C; Candlwic) at the bottom of an empty 6.5 cm diameter glass jelly jar. The wax was prepared by melting 73.6g of paraffin and soy wax mixture (Global Tech Industries, Cornelia, GA) in a stainless steel container at 80°C and adding 6.4g (8% by wt.) of fragrance oil while stirring. Once cooled to 70°C, the mixture was poured into the candle container and allowed to cool to a solid. The high-performance candles were prepared the same way except melting 79.2g of paraffin and soy wax and using 0.8g (1% by wt.) of fragrance oil.

A three-wick candle format was used for burn and emissions testing. The standard candle was prepared by placing 3 wicks (size 44-18-24C; Candlewic) at the bottom of an empty 10 cm diameter candle glass container. The wax was prepared by melting 383.64g of paraffin and soy wax mixture (Global Tech Industries, Cornelia, GA) in a stainless steel container at 80°C and adding 33.36 g fragrance oil (8% by wt). Once cooled to 70°C, the mixture was poured into the candle container. The candle was allowed to cool to room temperature and the wicks were cut to approximately 1 cm long. The high-performance candles were prepared the same way except melting 412.83g of paraffin and soy wax and using 4.17g (1% by wt.) of fragrance oil.

The results of these analyses indicated that the burn performance of the high-performance candle was better than the standard candle (Table 25). The flame height stayed at optimal height throughout the life of the candle, increasing the pool temperature and rate of consumption.

**TABLE 25**

| Measured Parameter | Candle Formulation/Dosage | |
|---|---|---|
| | Standard Caramel Dosage at 8% | High-performance Caramel Dosage at 1% |
| Average Rate of Consumption (g/hr) | 11.2 | 12.1 |
| Average Wax Pool Temperature (C°) | 73.4 | 76.8 |
| Average Flame height at 2 hours (inches) | 0.7 | 0.95 |
| Average Flame height at 4 hours (inches) | 0.9 | 1.15 |

In addition, the high-performance candle also had a more even rate of consumption over the life of the candle (Table 26).

**TABLE 26**

| Hour | Standard Caramel at 8% | High-performance Caramel at 1% |
|---|---|---|
| 4 | 13.6 | 11.6 |
| 8 | 12.8 | 11.0 |
| 12 | 14.1 | 13.5 |
| 16 | 12.2 | 13.4 |
| 20 | 8.9 | 11.0 |
| 24 | 7.5 | 11.2 |
| 28 | 9.1 | 12.8 |
| Average | 11.2 | 12.1 |
| Std Dev | 2.62 | 1.12 |

The High-performance candle produced less soot production and emissions in the form of benzene and naphthalene (Table 27).

**TABLE 27**

| Candle Formulation/ Dosage | Soot Index | Benzene Concentration (µg/L) | Naphthalene Concentration (µg/L) |
|---|---|---|---|
| Standard Caramel Dosage at 8% | 8.6 | 0.23 | 0.67 |
| High-performance Caramel Dosage at 1% | 3.6 | 0.10 | 0.06 |

Even though there the fragrance dosage was an eighth of the standard fragrance, the high-performance candle performed on par or better in intensity with a standard candle formula in a similar odor direction (Table 28).

**TABLE 28**

| Formula/ Dosage | Cold Intensity (0 poor to 9 excellent) | Burn Intensity (0 poor to 9 excellent) |
|---|---|---|
| Standard Berry Fragrance at 8% dosage | 7 | 5.5 |
| High-Performance Berry Fragrance at 1% dosage | 8 | 6.5 |
| Standard Woody Fragrance at 8% dosage | 6 | 6 |
| High-Performance Woody Fragrance at 1% dosage | 6 | 7 |
| Standard Caramel Fragrance at 8% dosage | 6.5 | 7 |
| High-Performance Caramel Fragrance at 1% dosage | 6 | 6 |

### Example 9: High-Performance Fragrances in a Shower Gel and Shampoo Applications

This example provides clear body wash formulations (Tables 29-31) that use approximately half or even less surfactant, yet still have acceptable viscosity, leathering properties and especially fragrance.

**TABLE 29**

| Formulation | Ingredient | % |
|---|---|---|
| 1 | Sodium lauryl ethoxy sulfate | 6 |
| | Cocamidopropyl betaine | 3 |
| | NaCl | 8 |
| | Fragrance "Hi impact shampoo" | 0.15 |
| | water | 82.85 |
| 2 | Sodium lauryl sulfate | 9 |
| | Cocamidopropyl betaine | 1 |
| | NaCl | 5 |
| | Fragrance "Hi impact shampoo" | 0.15 |
| | water | 84.85 |
| 3 | Sodium lauroyl sarcosinate | 7 |
| | Cocamide diethanolamine | 3 |
| | NaCl | 5 |
| | (EO)x(PO)y copolymer | 1 |
| | Fragrance "Hi impact shampoo" | 0.15 |
| | water | 83.85 |

In all cases of shower gels containing less than 10% surfactant, fragrances dosed above 0.4 wt% caused opacity, defined herein as a transmission % at 600 nm <80%. With properly selected aroma materials used to form a fragrance of high intensity, a lower level of fragrance can be used. Ideally, a shower gel or shampoo formulated from sustainable, biodegradable surfactant active compounds at low level with robust fragrance include: 8% Sodium lauryl Sarcosinate, 2% Cocobetaine, 3% NaCl, and 0.15**%** High Impact fragrance

When used in a shampoo application (Magick Botanicals shampoo), the fragrance does not exceed the solubilization capacity of the formula (which is reduced compared to conventional formulations because there is less surfactant). the lower level of fragrance leaves the liquid clear, although the aroma is intense and has good bloom characteristics (bloom defined as increased perception of fragrance during dilution, lathering and in general use)(Table 30).

**TABLE 30**

| | Characteristic | Fragrance Level | | | |
|---|---|---|---|---|---|
| | | 0 | 0.2 | 0.4 | 0.8 |
| High Impact 3 | transmission % 600nm | 100 | 100 | 4.1 | <1 |
| | viscosity [cP] | 2840 | 510 | 120 | 40 |
| Hi impact Shampoo | transmission % 600nm | 100 | 100 | 9.3 | <1 |
| | viscosity [cP] | 2840 | 1080 | 160 | 60 |
| Hi impact fabcon/shampoo | transmission % 600nm | 100 | 100 | 3.3 | <1 |
| | viscosity [cP] | 2840 | 1020 | 90 | 50 |
| Chypre type | transmission % 600nm | 100 | 95 | 2.9 | <1 |
| | viscosity [cP] | 2840 | 610 | 100 | 70 |
| Suavitel | transmission % 600nm | 100 | 100 | 70 | 2.5 |
| | viscosity [cP] | 2840 | 930 | 100 | 70 |
| Benchmark Fragrance 2 | transmission % 600nm | 100 | 100 | 4.3 | <1 |
| | viscosity [cP] | 2840 | 820 | 120 | 100 |
| Benchmark Fragrance 1 | transmission % 600nm | 100 | 100 | 16 | <1 |
| | viscosity [cP] | 2840 | 900 | 110 | 70 |
| Channel type | transmission % 600nm | 100 | 100 | 3.1 | <1 |
| | viscosity [cP] | 2840 | 720 | 110 | 60 |

### Example 10: High-Performance Fragrances in an Antiperspirant Roll-On Applications

*Deodorant Preparation.* The microcapsule slurry and the deodorant roll-on base were pre-mixed separately with an overhead mixer until homogeneous. The appropriate amount of microcapsule slurry was added to the roll-on base and mixed either by an overhead mixer or other mixing apparatus until homogeneous. The roll-on base containing the microcapsule was set aside at room temperature for at least 2 days prior to being evaluated.

*Sample Preparation.* The roll-on sample (0.30 - 0.35 gram) was applied to a 1.5-inch square area on a fragrance testing blotter (3 inches by 5 inches) and left to air out for 5 hours at room temperature. This was used for Pre-Activation evaluation. A similar blotter card was prepared at the same time for the Post-Activation evaluation sample. About 15-20 minutes prior to the evaluation session, a similar blotter card was prepared to serve as the Initial Application sample.

*Sample Evaluation.* The evaluation of microcapsule performance in a deodorant roll-on was conducted using an expert panel made up of 4-6 individuals very familiar with fragrance evaluations for deodorants. The panel of 5-6 experts was composed of 1-2 evaluators, 1-2 perfumers, and 1-2 product development scientists.

The Initial Application and Pre-Activation blotters cards were smelled first by the expert panel and each person assigned a rating (Table 31). The Post-Activation blotter card was folded in half and the sample area was sheared by moving the two halves of the blotter card in opposite directions 4-5 times. This action served to break the microcapsules, thereby releasing the fragrance core. The sheared card was assigned a rating by each person. The mean score was determined for each of the three evaluation stages.

*Rating*. A fragrance intensity index is used to rate the fragrance intensity in a fragrance composition or a consumer product containing the same (together referred to as "sample"). The fragrance intensity index is the ratio between (i) the sensory intensity score of a sample and (ii) the sensory intensity score of allyl amyl glycolate (AAG), as the standard. The sensory intensity score of AAG is scaled at a range of 0 to 100 evaluated by a sensory panel when AAG is dissolved in an appropriate solvent (e.g. diethyl phthalate) at a concentration of 0.015%. The sample is also evaluated by the same panel at a concentration of 0.015%, preferably under the same conditions and in the same sensory evaluation study conducted within the same day. A score of 5 means that the sample has a weak smell. A score of 15 indicates a medium smell. A score of 35 indicates a strong smell.

The fragrance intensity score is evaluated according to known industry protocols. See, *e.g.*, US20200046616A1 and US9162085B2. As an illustration, a personal wash product (or another consumer product such as fabric conditioner, detergent, all-purpose cleaner, shampoo, hair conditioner, etc.) is tested on a forearm (or a cloth, a hard surface, hair, etc., depending on the consumer product) using the following protocol: wet a forearm under running water (35 °C +/- 3 °C, 1.8 L/min) for 5 seconds; apply to the forearm 1 mL of the personal wash product, lightly wash the inner forearm for 10 seconds in a long, circular strokes with the opposite palm; allow 15 seconds residence time; rinse the forearm with running water for 15 seconds; dry the forearm with a clean cotton towel laying on the forearm while the opposite hand walks along the inner arm without rubbing; allow the forearm to dry in air for 30 seconds; evaluate the fragrance performance with a score of 1-100. In a simplified evaluation a score of 0-10 or 0-5 is used instead of 1-100. AAG is evaluated as a standard by applying AAG solution to the wet forearm with rinsing but not washing. The fragrance intensity index is then calculated as: sensory intensity score of a sample / sensory intensity score of AAG.

A fragrance composition or consumer product of this invention typically has a fragrance intensity index of at least 0.1, (e.g., at least 0.5, at least 1, 0.1 to 5, 0.2 to 4, 0.5 to 3, and 1.5).

Alternatively, a fragrance composition or consumer product of this invention especially a leave-on antiperspirant/deodorant is rated in a simplified evaluation with indications whether or not it is acceptable. A rating of " acceptable" for Initial Application and Pre-Activation corresponds to the intensity weaker than that assigned by an expert panel to a 0.75% dilution of allyl amyl glycolate (AAG) in diethyl phthalate. If the odor was stronger, a rating of "not acceptable" was assigned, which was considered inferior, an indication of fragrance leakage from the product before application to a treated surface. A rating of "acceptable" for Post-Activation if the fragrance intensity is equal to or greater than that of a 1.5% dilution of AAG in diethyl phthalate. If the odor is strong, a rating of "acceptable plus" is assigned. A sample with an odor intensity weaker than 1.5% dilution of AAG in diethyl phthalate is rated as "not acceptable" and considered inferior.

**TABLE 31**

| Intensity at Initial Application and at Pre-activation | Intensity upon Activation |
|---|---|
| Acceptable | Acceptable plus |
| Not acceptable | Acceptable |
| | Not acceptable |

### Example 11: Encapsulated High-Performance Fragrances in an Antiperspirant Roll-On Applications

*Fragrance Formulations*. A series of standard and High-Performance fragrance formulations were prepared (Table 32).

**TABLE 32**

| Fragrance | Accord A, wt% | Accord B, wt% | Accord C, wt% | Total, wt% A+B+C |
|---|---|---|---|---|
| Fougere Accord S1 | 2.1 | 4.4 | 28.3 | 34.8 |
| Standard | | | | |
| Fresh Accord S1 | 0.23 | 7.8 | 55.0 | 63.0 |
| Standard | | | | |
| Fruity Accord S1 | 1.1 | 0.3 | 27.3 | 28.7 |
| Standard | | | | |
| Fresh Accord U1 | 7.6 | 19.5 | 35.2 | 60.5 |
| High Performing | | | | |
| Amber Gourmand Accord U1 | 56.5 | 5.9 | 28.2 | 90.6 |
| High Performing | | | | |
| Fruity Accord U1 | 9.6 | 15 | 24 | 48.6 |
| High Performing | | | | |

*Antiperspirant Formulation.* An antiperspirant emulsion roll-on formulation was prepared (Table 33).

**TABLE 33**

| Ingredient | Wt% |
|---|---|
| Aluminum chlorohydrate | 10-16 |
| Emulsifiers, *e.g.*, Alkyl-PEG ethers such as Steareth-2, Steareth-20, Steareth-21, etc. | 3-7 |
| Humectant, *e.g.*, glycerin | 0-3 |
| Emollient, *e.g.,* mineral oil | 0-6 |
| Silica | 0.5-1 |
| Preservative | 0-1 |
| Water | Balance of formula |

*Experiment 1.* Microcapsules (capsule type PU-1) were prepared according to the method disclosed in US 2011/0071064 A1 using polyurea as the encapsulating polymer. The microcapsule aqueous suspension was added to an unfragranced antiperspirant roll-on base at a dosage sufficient to provide a fragrance neat oil equivalent (NOE) of either 0.5 wt% or 0.05 wt% in the roll-on base. The roll-on base samples with the microcapsules were allowed to equilibrate at least 2 days at room temperature before being evaluated by an expert panel according to the method described in Example 10.

The results of the evaluation are shown in Table 34. Polyurea microcapsules containing Standard or High-Performance fragrance performed similarly at initial application and met performance criteria. However, at 0.05 wt% NOE, only the microcapsule with High-Performance fragrance met the performance criteria on intensity upon activation.

**TABLE 34**

| Fragrance Type | Initial Application Intensity @ % 0.5 NOE | Initial Application Intensity @ 0.05% NOE | Intensity upon Activation @ 0.5% NOE | Intensity upon Activation @ 0.05% NOE |
|---|---|---|---|---|
| Standard Fougere Accord S1 | Acceptable | Acceptable | Acceptable | Not acceptable |
| High-Performance Fruity Accord U1 | Acceptable | Acceptable | Acceptable | Acceptable plus |

*Experiment 2.* Microcapsules and antiperspirant roll-on samples were prepared as described for Experiment 1. At 0.5 wt% NOE, polyurea capsules including a standard fragrance (Fresh Accord S1, PU-2) met the target residual levels for Type 1 Residuals. Polyurea capsules including a High-Performance fragrance (Fresh Accord U1, PU-1) met target residuals at less than or equal to 0.125 wt% NOE and met both of the performance criteria in antiperspirant roll-on (Table 35).

**TABLE 35**

| Capsule Type | Dosage %NOE | Type 1 Residuals¹ in AP roll-on | Initial Application Intensity | Intensity upon Activation |
|---|---|---|---|---|
| PU-2 | 0.5% | 1.25 micromol/g product Meets target | Acceptable | Acceptable plus |
| PU-1 | 0.5% | 2.5 micromol/g product Exceeds target | Not acceptable | Acceptable plus |
| | 0.125% | 0.625 micromol/g product Meets target | Acceptable | Acceptable |
| | 0.05% | 0.25 micromol/g product Meets target | Acceptable | Acceptable |

| | | | | |
|---|---|---|---|---|
| ¹Moles of primary amine groups in water-soluble (>5000 mg/L) polyamine molecules, max molecular weight of 1000 kDa. | | | | |

*Experiment 3.* Antiperspirant roll-on samples and polyurea microcapsules were prepared as in Experiment 1, except that the microcapsule core contained a high amount of a medium chain triglyceride as solvent. Hence, the dosage of the microcapsule aqueous suspension in the antiperspirant roll-on was 10 times more than the microcapsule wherein the core contained no solvent.

The results of the performance evaluation are shown in Table 36. The microcapsule with the High-Performance fragrance provided greater intensity upon activation compared to the Standard fragrance. Interestingly, the performance of the High-Performance fragrance on activation was also better, *i.e.,* more intense fragrance perceived, compared to the sample wherein the microcapsule core did not contain any solvent.

**TABLE 36**

| Fragrance Type* | Microcapsule Core Solvent to Fragrance Ratio wt/wt | Intensity before Activation | Intensity upon Activation |
|---|---|---|---|
| Standard Fresh Accord S1 | 90/10 | Acceptable | Acceptable |
| High-Performance Fresh Accord U1 | 90/10 | Acceptable | Acceptable plus |
| | 0/100 | Acceptable | Acceptable |

| | | | |
|---|---|---|---|
| *All fragrances used a dosage of 0.05% NOE. | | | |

*Experiment 4.* A High-Performance fragrance (Amber Gourmand U1) was created that contained several fragrance materials that have a potential to undergo a color change in antiperspirant roll-on under accelerated storage conditions. A microcapsule aqueous suspension was created as described for Experiment 1, using the Amber Gourmand U1 fragrance. Antiperspirant roll-on samples were prepared and changes in color were monitored. As shown in Table 37, color change was avoided in the antiperspirant roll-on when the High-Performance fragrance was encapsulated in microcapsules and dosed in the antiperspirant roll-on even at the highest dosage of 0.30 wt% NOE.

**TABLE 37**

| Sample in AP Roll-on | Dosage %NOE | AP Roll-on Color 2 weeks at 50°C |
|---|---|---|
| Unfragranced Control AP Roll-on Base | 0.0 | Acceptable |
| Amber Gourmand U1 Fragrance Only | 0.30 | Not Acceptable Brownish Yellow |
| Amber Gourmand U1 Fragrance Only | 0.15 | Not Acceptable Light Brownish Yellow |
| Amber Gourmand U1 Fragrance Only | 0.05 | Borderline Acceptable Light Creamy Yellow |
| Amber Gourmand U1 PU-1 Capsule | 0.30 | Acceptable Similar to Control |
| Amber Gourmand U1 PU-1 Capsule | 0.15 | Acceptable Similar to Control |
| Amber Gourmand U1 PU-1 Capsule | 0.05 | Acceptable Similar to Control |

Performance and olfactive evaluations of the same High-Performance fragrances dosed in antiperspirant roll-on at 0.01 wt% to 0.30 wt% NOE are presented in Table 38. The olfactive profile was more hedonically appealing at the lower NOE and met the performance criteria at dosages less than or equal to 0.15 wt% NOE, and more surprisingly met all the performance criteria even at 0.01 wt% NOE. The olfactive profile dynamically transitioned from an aldehydic, clean fresh floral, slight amber at 0.01 wt% NOE to a heavy amber, less fresh floral at 0.03 wt% NOE.

**TABLE 38**

| Dosage* %NOE | Intensity Before Activation | Intensity Upon Activation | Olfactive Description upon Activation |
|---|---|---|---|
| 0.01% | Acceptable | Acceptable | Mandarin aldehydic, very clean, fresh floral, woody amber back |
| 0.05% | Acceptable | Acceptable | Slightly more woody amber; still very floral/fresh/aldehydic |
| 0.15% | Acceptable | Acceptable plus | Significantly more woody amber; subtle cilantro note |
| 0.30% | Not acceptable | Acceptable plus | Very heavy amber, less fresh/floral |

| | | | |
|---|---|---|---|
| *Polyurea encapsulated High-Performance Amber Gourmand U1 fragrance dose. | | | |

*Experiment 5.* One of the performance criteria for a microcapsule is to have minimal distortion of the neat oil (parent fragrance) upon initial application. Hence, antiperspirant roll-on samples were prepared (Table 39) as described for Experiment 1 with and without polyurea microcapsules to determine if the presence of two different High-Performance fragrances might distort the olfactive character of the neat oil on initial application.

**TABLE 39**

| Sample | Neat Oil ID | Neat Oil (%) | Capsule Fragrance ID | Fragrance Type | Capsule Type | Capsule Dosage % NOE |
|---|---|---|---|---|---|---|
| **1** | Male Herbal S1 | 1.4 | N/A | Standard | N/A | N/A |
| **2** | Male Herbal S1 | 1.4 | Fresh Accord U1 | High-Performance | PU-1 | 0.15 |
| **3** | Male Herbal S1 | 1.4 | Fresh Accord U1 | High-Performance | PU-1 | 0.05 |
| **4** | Male Herbal S1 | 1.4 | Fresh Accord U1 | High-Performance | PU-1 | 0.01 |
| **5** | Female Floral S1 | 1.3 | N/A | Standard | N/A | N/A |
| **6** | Female Floral S1 | 1.3 | Amber Gourmand U1 | High-Performance | PU-1 | 0.15 |
| **7** | Female Floral S1 | 1.3 | Amber Gourmand U1 | High-Performance | PU-1 | 0.05 |
| **8** | Female Floral S1 | 1.3 | Amber Gourmand U1 | High-Performance | PU-1 | 0.01 |

A standard odor descriptor lexicon was used to describe the olfactive impression of the expert panel at each of the different performance criteria (Table 40).

**TABLE 40**

| Sample | Initial Application (A) | Pre-Activation (5 hrs Post Application) (B) | Post-Activation (5 hrs Post Application) (C) |
|---|---|---|---|
| **1** | Tobacco, musk, woody, herbal, spice | Same as 1(A) | Tobacco, musk, amber, woody, herbal, spice |
| **2** | Same as 1A but slightly more woody and musky | Same as 1(A) but slightly more herbal | Violet, green galbanum, amber |
| **3** | Same as 1A | Same as 1(A) but slightly more musky | Same as 2(C) but slightly less violet, more orris, amber, woody |
| **4** | Same as 1A | Same as 1(A) but slightly more musky | Same as 3(C) but more violet, less orris, woody |
| **5** | Jasmine, green grassy, aldehyde, fruity | Same as 5(A) | Green grassy, floral |
| **6** | Same as 5(A) but slightly less aldehydic, more woody, amber | Same as 5(A) | Green grassy, aldehydic, woody, amber, cassis |
| **7** | Same as 5(A) but slightly less aldehydic | Same as 5(A) | Same as 6(C) but more aldehydic, green cilantro-like, less woody |
| **8** | Same as 5(A) but slightly less aldehydic | Same as 5(A) but slightly more aldehydic | Same as 6(C) but more floral, musky, creamy woody |

As indicated in Table 40, there was very little to no distortion of the neat oil on initial application and even at pre-activation. At post-activation, the High-Performance fragrance was released from the microcapsules and the olfactive character changed such that it was a harmonious blend of the High-Performance fragrance and residual fragrance notes remaining from the neat oil after 5 hours. Surprisingly, at the capsule dosage of 0.01 wt% NOE, a noticeable olfactive change was still readily perceived after post-activation.

*Experiment 6.* Silica microcapsules were prepared according to the method disclosed in US 9,044,732 B2. The silica microcapsule aqueous suspension was added to an unfragranced antiperspirant roll-on base at a dosage sufficient to provide a fragrance neat oil equivalent (NOE) of either 0.5 wt% or 0.05 wt% in the antiperspirant roll-on base. The antiperspirant roll-on base samples with the silica microcapsules were allowed to equilibrate at least 2 days at room temperature before being evaluated by an expert panel according to the method described in Example 10.

The results of the performance evaluation are summarized in Table 41. Microcapsules containing Standard (Fougere Accord S1) and High-Performance (Fruity Accord U1) fragrance performed similarly at Initial Application and met performance criteria. However, at 0.05 wt% NOE, the microcapsule with High-Performance fragrance had higher intensity upon activation compared to the standard fragrance.

**TABLE 41**

| Fragrance Type | Initial Application Intensity @ % 0.5 NOE | Initial Application Intensity @ 0.05% NOE | Intensity upon Activation @ 0.5% NOE | Intensity upon Activation @ 0.05% NOE |
|---|---|---|---|---|
| Standard | Acceptable | Acceptable | Acceptable plus | Acceptable |
| High-Performance | Acceptable | Acceptable | Acceptable plus | Acceptable plus |

*Experiment 7.* Antiperspirant roll-on samples were prepared as in *Experiment 6*. Silica microcapsules were prepared similarly as in *Experiment 6* except that the microcapsule core may contain a high amount of a medium chain triglyceride as solvent. Hence, the dosage of the microcapsule aqueous suspension in the antiperspirant roll-on was 10 times more for the microcapsule wherein the core has a solvent to fragrance ratio of 90/10 compared to the microcapsule core that contained no solvent (0/100).

At similar NOE, no difference was observed in performance for intensity before and after activation for Silica microcapsules with Standard fragrance whether the fragrance core was with or without solvent (Table 42). The same applied with the High-Performance fragrance. However, the latter outperformed the Standard fragrance on intensity upon activation at either 0.05 wt% or 0.30 wt% NOE in antiperspirant roll-on.

**TABLE 42**

| Fragrance Type | Dosage %NOE | Microcapsule Core Solvent to Fragrance Ratio wt/wt | Intensity before Activation | Intensity upon Activation |
|---|---|---|---|---|
| Standard Fruity Accord S1 | 0.30 | 0/100 | Not acceptable | Acceptable |
| Standard Fruity Accord S1 | 0.30 | 90/10 | Not acceptable | Acceptable |
| Standard Fruity Accord S1 | 0.05 | 0/100 | Acceptable | Not acceptable |
| Standard Fruity Accord S1 | 0.05 | 90/10 | Acceptable | Not acceptable |
| High-Performance Fruity Accord U1 | 0.30 | 0/100 | Not acceptable | Acceptable plus |
| High-Performance Fruity Accord U1 | 0.30 | 90/10 | Not acceptable | Acceptable plus |
| High-Performance Fruity Accord U1 | 0.05 | 0/100 | Acceptable | Acceptable plus |
| High-Performance Fruity Accord U1 | 0.05 | 90/10 | Acceptable | Acceptable plus |

### Example 12: Malodor Coverage

The malodor coverage properties of the Standard (Fresh Accord S1) and High-Performance (Fresh Accord U1) fragrances versus a Sweat Malodor Model were determined according US 9,737,628 B2, incorporated herein by reference in its entirety. Data were analyzed using Three-Way ANOVA (JMP Fit Model) and Post-Hoc with Tukey Multiple Comparisons.

As shown in Table 43, sweat malodor intensity was significantly lower for the High-Performance fragrance. Moreover, the perception of sweat malodor was significantly greater for the Standard fragrance at 0.3 wt% compared to 0.05 wt% of the High Performing fragrance.

**TABLE 43**

| Fragrance Dosage Wt% in triethyl citrate | Malodor Intensity (LMS Scale) vs. Sweat Model (N=20) | Standard Error | Post-Hoc Result* |
|---|---|---|---|
| 0.30¹ | 10.33 | 1.08 | c |
| 0.15¹ | 10.91 | 1.08 | bc |
| 0.05¹ | 13.19 | 1.05 | ab |
| 0.30² | 4.51 | 1.14 | e |
| 0.15² | 5.04 | 1.13 | e |
| 0.05² | 7.39 | 1.11 | d |

| | | | |
|---|---|---|---|
| *Letters that are different indicates that the samples are significantly different from each other (p ≤ 0.05). 1 Standard fragrance Fresh Accord S1 2 High-Performance fragrance Fresh Accord U1 | | | |

Two separate paired comparison tests were conducted. The sensory panel was asked to select the sample from each pair that had more sweat malodor. The results are shown in Table 44. No difference was found between two Standard fragrances at 0.3 wt% vs. two High-Performance fragrances at 0.05 wt% indicating good malodor coverage for the latter in spite of being about 6x lower concentration.

| **Paired Comparison Sample Pair** | **N** | **# choosing sample with more malodor** | **Result*** |
|---|---|---|---|
| 0.3 wt% Fresh Accord S1 (Standard Fragrance) vs. | 20 | 13 vs. 7 | No significant difference |
| 0.05 wt% Fresh Accord U2 (High Performing Fragrance) | | | |
| 0.3 wt% Fruity Accord S1 (Standard Fragrance) vs. | 19 | 10 vs. 9 | No significant difference |
| 0.05 wt% Fruity Accord U2 (High Performing Fragrance) | | | |

| | | | |
|---|---|---|---|
| *Critical number of correct responses in a two-sided directional difference test for significance at p=0.05 is 15. | | | |

## Claims

1. A consumer product with an improved aesthetic comprising
(i) ≤ 1% of a High-Performance fragrance composition and
(ii) a consumer product active,
wherein the High-Performance fragrance composition comprises at least 55% by weight of one or more of High-Performance fragrance ingredients selected from the group consisting of the High-Performance fragrance ingredients listed in Table 2, and
wherein Table 2 is
| Fragrance | Chemical Name | CAS No. |
|---|---|---|
| Abhexone | 2~{H}-furan-5-one, 2-ethyl-4-hydroxy-3-methyl- | 698-10-2 |
| AGRUNITRILE | 6-octenenitrile, 3,7-dimethyl- | 51566-62-2 |
| Amberketal 8.5 Pct IPM | 14,16-dioxatetracyclo[11.2.1.0^{1,10}.0^{4,9}]hexade cane, 5,5,9,13-tetramethyl- | 57345-19-4 |
| Amber Xtreme (ELINCS) | 3,3a,4,5,5a,7,8a,8b-octahydrocyclopenta[g]benzofuran, (3aS,8bS)-2,2,6,6,7,8,8-heptamethyl- | 476332-65-7 |
| AMBRINOL 95 PRG | 1,3,4,4~{a},6,7-hexahydronaphthalen-2-ol, 2,5,5-trimethyl- | 41199-19-3 |
| ALD C-16 STRAWB#2 | 2-oxiranecarboxylic acid, (2S,3R)-3-methyl-3-phenyl-, ethyl ester | 19464-92-7 |
| ACETOPHENONE | ethanone, 1-phenyl- | 98-86-2 |
| Acetyl 2-, Pyridine 10% IPM | ethanone, 1-(2-pyridinyl)- | 1122-62-9 |
| ALD AA TRIPLAL BHT | 1-cyclohex-3-enecarboxaldehyde, (1S,2R)-2,4-dimethyl- | 188716-52-1 |
| Ald C-6 Toco | hexanal | 66-25-1 |
| ALD C-8 TOCO | octanal | 124-13-0 |
| ALD C-10 | decanal | 112-31-2 |
| ALD C-9 TOCO | nonanal | 124-19-6 |
| ALD C-11 ULENIC TOCO | 10-undecenal | 112-45-8 |
| ALD C-11 UNDECYLIC TOCO | undecanal | 112-44-7 |
| ALD C-12 LAURIC TOCO | dodecanal | 112-54-9 |
| ALD C-18 | 2-oxolanone, 5-pentyl- | 104-61-0 |
| MELOZONE | 3-tricyclo[5.2.1.02,6]decanecarboxaldehyde, (1S,2R,6S,7R)- | 30772-79-3, 91967-77-0 |
| AMBROCENIDE CRYST | 4,6-dioxatetracyclo[6.5.1.01,10.03,7]tetradecane, 5,5,7,9,9,13-hexamethyl- | 211299-54-6 |
| AMBRETTOLIDE | 1-oxacycloheptadec-10-en-2-one, (10~{E})- | 63286-42-0 |
| AMBERMOR EX | 2,4,5,5~{a},7,8,9,9~{b}-octahydro-1~{H}-benzo[e]benzofuran, (3~{a}~{S},5~{a}~{R},9~{a}~{R},9~{b}~{S})-3~{a},6,6,9~{a}-tetramethyl- | 3738-00-9, 6790-58-5 |
| ACETYL METH CARBINOL 10% DPG | 2-butanone, 3-hydroxy- | 513-86-0 |
| BENZALD FFC 10% DPG | benzaldehyde | 100-52-7 |
| CALONE | 1,5-benzodioxepin-3-one, 7-methyl- | 28940-11-6 |
| CASSIS ETHER IPM | tetradecanoic acid, propan-2-yl ester | 110-27-0 |
| CASHMERAN | 2,5,6,7-tetrahydroinden-4-one, 1,1,2,3,3-pentamethyl- | 33704-61-9 |
| Corps Pamplemousse Pure | 2-propanethiol, 2-(4-methyl-1-cyclohexenyl)- | 61758-19-8 |
| CYCLEMAX | propanal, 3-(4-propan-2-ylphenyl)- | 7775-00-0 |
| CEDRAMBER | tricyclo[5.3.1.0^{1,5}]undecane, (1~{S},2~{R},5~{S},8~{R})-8-methoxy-2,6,6,8-tetramethyl- | 67874-81-1 |
| CITRAL NEW | octa-2,6-dienal, (2E)-3,7-dimethyl- | 141-27-5 |
| Citrylal | benzene, 1-methyl-4-propan-2-yl- | 99-87-6 |
| Clonal | dodecanenitrile | 2437-25-4 |
| Corps Racine Vs 600164 Conc | pyridine, 2-(3-phenylpropyl)- | 2110-18-1 |
| Costasid | 2-octenoic acid, 4-ethyl- | 90464-78-1 |
| CREOSOL #340 | phenol, 2-methoxy-4-methyl- | 93-51-6 |
| CYCLAPROP | propanoic acid, 8-tricyclo[5.2.1.0^{2,6}]dec-4-enyl ester | 67634-24-6 |
| Dimethyl Sulfide Nat | methane, (methylthio)- | 75-18-3 |
| Decenal, 9, | 9-decenal | 39770-05-3 |
| Dimeth Phenol,2,3, | phenol, 2,3-dimethyl- | 526-75-0 |
| Dimeth Phenol,2,4, | phenol, 2,4-dimethyl- | 105-67-9 |
| DORIFFOX | oxane, (2~{S},4~{R})-4-methyl-2-phenyl- | 149713-23-5, 149713-24-6, 94201-73-7 |
| DODECENAL TRANS-2 TOCO | 2-dodecenal, (~{E})- | 20407-84-5 |
| OPALENE BHT | 8-decenal, (~{E})- | 174155-47-6 |
| DECENAL,2, TOCO | 2-decenal, (~{E})- | 3913-81-3 |
| DECENAL,CIS-4 | 4-decenal, (~{Z})- | 21662-09-9 |
| DELPHONE | 1-cyclopentanone, 2-pentyl- | 4819-67-4 |
| DAMASCONE DELTA | 2-buten-1-one, (E)-1-[(1S,2R)-2,6,6-trimethyl-1-cyclohex-3-enyl]- | 71048-82-3 |
| DIPHEN OXIDE | benzene, phenoxy- | 101-84-8 |
| DUPICAL NAAR | butanal, 4-(8-tricyclo[5.2.1.02,6]decanylidene)- | 30168-23-1 |
| EUGENOL NAT EX CLOVE LEAF OIL | phenol, 2-methoxy-4-prop-2-enyl- | 97-53-0 |
| TROPICALIA | 2-bicyclo[2.2.1]hept-5-enecarboxylic acid, (2~{R})-, ethyl ester | 51789-95-8 |
| ETH ACETO ACET 10% DPG | butanoic acid, 3-oxo-, ethyl ester | 141-97-9 |
| ETH CAPROATE | hexanoic acid, ethyl ester | 123-66-0 |
| ETH-2-METH BUTY | butanoic acid, 2-methyl-, ethyl ester | 7452-79-1 |
| ETH PHEN GLYC | 2-oxiranecarboxylic acid, (2~{S},3~{R})-3-phenyl-, ethyl ester | 2272-55-1 |
| ETH SAL | benzoic acid, 2-hydroxy-, ethyl ester | 118-61-6 |
| EUCALYPTOL USP | 2-oxabicyclo[2.2.2]octane, 1,3,3-trimethyl- | 470-82-6 |
| FILBERTONE 22350 | 2-hepten-4-one, (~{E})-5-methyl- | 102322-83-8 |
| STARFLEUR TOCO | propanal, 3-[4-(2-methylpropyl)cyclohexyl]- | 1315250-65-7 |
| FRUITATE (ELINCS) | 2-tricyclo[5.2.1.0^{2,6}]decanecarboxylic acid, ethyl ester | 129520-41-8 |
| FLORAL SUPER | deca-4,9-dienal, (4E)-4,8-dimethyl- | 77016-39-8 |
| FLORHYDRAL TOCO (ELINCS) | butanal, 3-(3-propan-2-ylphenyl)- | 125109-85-5 |
| FRAGARONE | 2-pentenoic acid, (~{E})-2-methyl- | 16957-70-3 |
| Galbazine | pyrazine, 2-methoxy-3-(2-methylpropyl)- | 24683-00-9 |
| Geosmin Neat | 1,2,3,4,5,6,7,8-octahydronaphthalen-4~{a}-ol, 4,8~{a}-dimethyl- | 19700-21-1 |
| GALBASCONE | 4-penten-1-one, 1-(5,5-dimethyl-1-cyclohexenyl)- | 56973-85-4 |
| Ald C-7 Stabiliff | heptanal | 111-71-7 |
| HEALINGWOOD | | 5986-55-0 |
| METH DH JASMONATE | acetic acid, 2-[(1~{S},2~{S})-3-oxo-2-pentylcyclopentyl]-, methyl ester | 133163-97-0, 2570-03-8, 29852-02-6 |
| HEXADECANOLIDE | oxacycloheptadecan-2-one | 109-29-5 |
| Homo Pineapple CMPD | 3-furanone, 2-ethyl-4-hydroxy-5-methyl- | 27538-10-9 |
| ISO VALERIC ACID 0.1% DPG | butanoic acid, 3-methyl- | 503-74-2 |
| ALD C-11 INTRELEVEN (TT) PRG | 9-undecenal, (~{E})- | 143-14-6 |
| IRONE V BHT 1% DPG | 3-buten-2-one, (~{E})-4-[(1~{S},5~{R})-2,5,6,6-tetramethyl-1-cyclohex-2-enyl]- | 79-69-6 |
| Thiazole (4-Iso Propyl 2-Methyl) | thiazole, 4-methyl-2-propan-2-yl- | 15679-13-7 |
| IONONE ALPHA BHT | 3-buten-2-one, (~{E})-4-(2,6,6-trimethyl-1-cyclohex-2-enyl)- | 31798-11-5 |
| ORRIS ALD | 2-nonenal, (~{E})- | 18829-56-6 |
| ISO BUTYL QUINOLINE | quinoline, 6-butan-2-yl- | 65442-31-1 |
| ISO CYCLO CITRAL BHT | 1-cyclohex-3-enecarboxaldehyde, (1~{R},2~{S},6~{S})-2,4,6-trimethyl- | 1423-46-7 |
| Iso Propyl Quinoline | quinoline, 6-propan-2-yl- | 135-79-5 |
| Iso Valeric Ald | butanal, 3-methyl- | 590-86-3 |
| JAVANOL TT (ELINCS) | methanol, [1-methyl-2-[(1,2,2-trimethyl-3-bicyclo[3.1.0]hexanyl)methyl]cyclopropyl]- | 198404-98-7 |
| KOUMALACTONE 10 PCT TEC FIRM | 3a,4,5,6,7,7a-hexahydro-3H-benzofuran-2-one, 3,6-dimethyl- | 79726-51-5, 92015-65-1 |
| KHUSINIL (ELINCS) | pentanenitrile, 2,2-dimethyl-4-phenyl- | 75490-39-0 |
| LACTONE OF CIS JASMONE TOCO | 2-oxolanone, 5-[(~{Z})-hex-3-enyl]-5-methyl- | 70851-61-5 |
| Limoxal (Limonene Oxo Ald) | butanal, 3-(4-methyl-1-cyclohex-3-enyl)- | 50450-53-8, 6784-13-0 |
| LEMONILE | nona-2,6-dienenitrile, (2~{Z},6~{Z})-3,7-dimethyl- | 61792-11-8 |
| Undecatriene Super LRG 1218 RD BHT | undeca-1,3,5-triene, (3~{E},5~{E})- | 16356-11-9 |
| METH PAMPLEMOUSSE TOCO | 2-hexene, 6,6-dimethoxy-2,5,5-trimethyl- | 67674-46-8 |
| Maritima | pyridine, 4-[(3~{E})-4,8-dimethylnona-3,7-dienyl]- | 69511-23-5 |
| MANDARIL (ELINCS) BHT | trideca-3,12-dienenitrile, (3~{E})- | 134769-33-8 |
| MONTAVERDI | cyclopropanecarboxylic acid, [(~{Z})-hex-3-enyl] ester | 16428-99-2, 188570-78-7 |
| MUSCEMOR (ELINCS) | 1-cyclopentadec-5-enone, 3-methyl- | 63314-79-4 |
| METH JASMONATE TOCO | acetic acid, 2-[(1~{R},2~{S})-3-oxo-2-[(~{E})-pent-2-enyl]cyclopentyl]-, methyl ester | 95722-42-2 |
| MENTHONE 85 | 1-cyclohexanone, (2S,5R)-5-methyl-2-propan-2-yl- | 1074-95-9, 14073-97-3, 89-80-5 |
| METH LAITONE 10 PCT DPG | 1-oxaspiro[4.5]decan-2-one, 8-methyl- | 94201-19-1 |
| VERIDIAN | 3-decen-5-one, (~{E})-4-methyl- | 811412-48-3 |
| METH BENZOATE | benzoic acid, methyl ester | 93-58-3 |
| METH BUTYRIC ACID,2, | butanoic acid, 2-methyl- | 116-53-0 |
| METH CINNAMATE TOCO | 2-propenoic acid, (~{E})-3-phenyl-, methyl ester | 1754-62-7 |
| Jamunate | 1-cyclohexanecarboxylic acid, 3-methyl-, methyl ester | 72903-23-2 |
| MELONAL TOCO | 5-heptenal, 2,6-dimethyl- | 106-72-9, 77787-60-1 |
| Meth Heptin Carbonate | 2-octynoic acid, methyl ester | 111-12-6 |
| METH HEPTYL KETONE | 2-nonanone | 821-55-6 |
| ALD C-12 MNA TOCO | undecanal, 2-methyl- | 110-41-8 |
| METH OCTIN CARBONATE | 2-nonynoic acid, methyl ester | 111-80-8 |
| ALD C-11 MOA BHT | decanal, 2-methyl- | 19009-56-4 |
| METH PHEN ETH ETHER | benzene, 2-methoxyethyl- | 3558-60-9 |
| METH TUBERATE RD | 2-oxolanone, 4-methyl-5-pentyl- | 33673-62-0 |
| MANGONE | 1-cyclohexanone, (2~{S},5~{S})-2-(2-mercaptopropan-2-yl)-5-methyl- | 33281-91-3 |
| CRISTALFIZZ | butanal, 4-heptoxy-3-methyl- | 1093653-57-6 |
| AQUAFLORA TOCO | acetaldehyde, 2-[(1S,2R,6R,7S,8R)-8-tricyclo[5.2.1.02,6]decanyl]- | 1339119-15-1 |
| Pomelene 0.01Pct Ipm | 6-thiabicyclo[3.2.1]octane, 4,7,7-trimethyl- | 68398-18-5 |
| METH PARA CRESOL | benzene, 1-methoxy-4-methyl- | 104-93-8 |
| MUSCENONE (ELINCS) | 1-cyclopentadec-5-enone, 3-methyl- | 63314-79-4 |
| MYRAC ALD BHT | 1-cyclohex-3-enecarboxaldehyde, 4-(4-methylpent-3-enyl)- | 37677-14-8 |
| VERTONIC FOR NON TSCA USE ONLY | 3,6-dihydro-2~{H}-pyran, 4,6-dimethyl-2-(1-phenylethyl)- | 1945993-03-2 |
| NECTAROL SA | 1-cyclohex-2-enone, 3,5-diethyl-2,5-dimethyl- | 39121-42-1 |
| NECTARYL LRG | 1-cyclopentanone, 2-[2-(4-methyl-1-cyclohex-3-enyl)propyl]- | 95962-14-4 |
| Nonenol,Cis-6 Toco | 6-nonen-1-ol, (~{Z})- | 35854-86-5 |
| NONADIENAL 2-6 DIETH ACETAL | nona-2,6-diene, (2~{E},6~{Z})-1,1-diethoxy- | 67674-36-6 |
| Nonenal,Cis-6 Toco | 6-nonenal, (~{Z})- | 2277-19-2 |
| OXANE 50 PCT TEC | 1,3-oxathiane, (2~{R},4~{S})-2-methyl-4-propyl- | 59323-76-1 |
| OPERANIDE (ELINCS) | 4,5,7,8b-tetrahydro-3aH-cyclopenta[e][1,3]benzodioxole, 2,2,6,6,7,8,8-heptamethyl- | 823178-41-2 |
| ORENYLE | 2-nonenenitrile, (~{E})- | 40856-16-4 |
| Pyrazine,2-Eth-3,5-Dimeth | pyrazine, 2-ethyl-3,5-dimethyl- | 13925-07-0 |
| Pharaone 10 PCT DPG | 3-hepta-1,6-dienone, 2-cyclohexyl- | 313973-37-4 |
| MALTOL (PALATONE) | 4-pyranone, 3-hydroxy-2-methyl- | 118-71-8 |
| CRESETAL PARA | oxane, 2-(4-methylphenoxy)- | 13481-09-9 |
| CRESOL PARA EXTRA | phenol, 4-methyl- | 106-44-5, 1319-77-3 |
| Cresyl Acet Para Coeur | acetic acid, (4-methylphenyl) ester | 140-39-6 |
| Meth Quinoline Para | quinoline, 6-methyl- | 91-62-3 |
| PASSION FRUIT COMPOUND | | |
| PINEAPPLE CMPD 1% IPM (MB) | 3-furanone, 4-hydroxy-2,5-dimethyl- | 3658-77-3 |
| PATCHOULI OIL LIGHT BLO | 3-tricyclo[5.3.1.03,8]undecanol, 2,2,6,8-tetramethyl- | 5986-55-0 |
| PENTYL FURAN,2, | furan, 2-pentyl- | 3777-69-3 |
| Phenacetic Acid Extra (USDEA) | acetic acid, 2-phenyl- | 103-82-2 |
| PHEN ETH ACET | acetic acid, 2-phenylethyl ester | 103-45-7 |
| PINO ACETALD TOCO | propanal, 3-(6,6-dimethyl-2-bicyclo[3.1.1]hept-2-enyl)- | 33885-51-7 |
| POPCORN CHEMICAL | 5,7-dihydrothieno[3,4-d]pyrimidine, 2-methyl- | 36267-71-7 |
| PYRAZINE PM517 2-B-3-MEOXY | pyrazine, 2-butan-2-yl-3-methoxy- | 24168-70-5 |
| ROSYRANE SUPER | oxane, 4-methylene-2-phenyl- | 60335-74-2 |
| Robustone | furan, 2-[(2-furanylmethyldisulfanyl)methyl]- | 4437-20-1 |
| ROSALVA | 9-decen-1-ol | 13019-22-2 |
| ROSE OXIDE TOCO | oxane, (2~{R},4~{S})-4-methyl-2-(2-methylprop-1-enyl)- | 3033-23-6 |
| ROSETHYL | benzene, 1-(ethoxymethyl)-2-methoxy- | 64988-06-3 |
| Safranal Toco | 1-cyclohexa-1,3-dienecarboxaldehyde, 2,6,6-trimethyl- | 116-26-7 |
| SINENSAL NATURAL 20 EX ORANGE | dodeca-2,6,11-trienal, (2E,6E)-2,6-dimethyl-10-methylene- | 3779-62-2 |
| OCEANOL | benzoic acid, 2,4-dihydroxy-3-methyl-, methyl ester | 33662-58-7 |
| SAFRALEINE | 2~{H}-inden-1-one, 2,3,3-trimethyl- | 54440-17-4 |
| Spirogalbanone 10% DEP | 4-penten-1-one, 1-(9-spiro[4.5]dec-9-enyl)- | 224031-71-4 |
| Sacrazole-018 | ethanol, 2-(4-methyl-5-thiazolyl)- | 137-00-8 |
| SINFONIDE | 6,6a,8,9a-tetrahydro-5H-cyclopenta[h]quinazoline, (6aR,9aR)-7,7,8,9,9-pentamethyl- | 1356400-59-3 |
| TOFFEE LACTONE 2067 | 2-oxolanone, 5-hexyl- | 107797-26-2, 706-14-9 |
| TRIFERNAL BHT 10% DPG | butanal, 3-phenyl- | 16251-77-7 |
| COOLWOOD | 8-tricyclo[5.2.1.02,6]decanol, 5,7,8-trimethyl- | 1340502-69-3 |
| TERPINOLENE P UB BHT | cyclohexene, 1-methyl-4-propan-2-ylidene- | 586-62-9 |
| Pyrazine 004 (2-Ethyl-3-Meth) | pyrazine, 2-ethyl-3-methyl- | 15707-23-0 |
| Pyrazine 044 (Methyl) | pyrazine, 2-methyl- | 109-08-0 |
| Pyrazine 068 (2,6-Dimeth) | pyrazine, 2,6-dimethyl- | 108-50-9 |
| Pyrazine 043 (2,5-Dimeth) | pyrazine, 2,5-dimethyl- | 123-32-0 |
| Pyrazine 040 (2,3-Dimeth) | pyrazine, 2,3-dimethyl- | 5910-89-4 |
| Pyrazine 001 (2,3,5-Trimeth) | pyrazine, 2,3,5-trimethyl- | 14667-55-1 |
| Pyrazine 014 (Cyclo Hexa) | 5,6,7,8-tetrahydroquinoxaline | 34413-35-9 |
| Iso Butyl Thiazole | thiazole, 2-(2-methylpropyl)- | 18640-74-9 |
| TONKALACTONE | 3a,4,5,6,7,7a-hexahydro-3H-benzofuran-2-one, 3-ethyl- | 54491-17-7 |
| NONADIENAL,2-TR-6-CIS TOCO | nona-2,6-dienal, (2~{E},6~{Z})- | 557-48-2 |
| Nonadienol,2-Trans-6-Cis Toco | 1-nona-2,6-dienol, (2~{E},6~{Z})- | 28069-72-9 |
| DECENAL,TRANS 4, TOCO | 4-decenal, (~{E})- | 65405-70-1 |
| HEXENAL,TRANS 2, | 2-hexenal, (~{E})- | 6728-26-3 |
| Tridecene-1-Al,2, Toco | 2-tridecenal, (~{E})- | 7069-41-2 |
| Tridecene-2-nitrile | 2-tridecenenitrile, (~{E})- | 22629-49-8 |
| UNDECALACTONE,D ELTA | 2-oxanone, 6-hexyl- | 710-04-3 |
| ULTRAVANIL Q COLIPA | phenol, 2-ethoxy-4-methyl- | 2563-07-7 |
| UNDECAVERTOL TOCO | 3-decen-5-ol, (~{E})-4-methyl- | 81782-77-6 |
| VANITROPE | phenol, 2-ethoxy-5-[(~{E})-prop-1-enyl]- | 94-86-0 |
| VIONIL NEAT | nona-2,6-dienenitrile, (2~{E},6~{Z})- | 97752-28-8 |
| VARAMOL-106 | phenol, 4-ethenyl-2-methoxy- | 7786-61-0 |
| VELTOL PLUS | 4-pyranone, 2-ethyl-3-hydroxy- | 4940-11-8 |
| VERAMOSS | benzoic acid, 2,4-dihydroxy-3,6-dimethyl-, methyl ester | 4707-47-5 |
| Verdima | pyridine, 2-pentan-3-yl- | 7399-50-0 |
| VERDURAL B EXTRA | propanoic acid, 2-methyl-, [(~{Z})-hex-3-enyl] ester | 41519-23-7 |
| Verdoracine | benzene, 1-methyl-4-propan-2-yl-2-[(~{E})-prop-1-enyl]- | 14374-92-6 |
| VETIVERT ACET HAITI BLO | acetic acid, [(1~{R},5~{S})-7,7-dimethyl-6-methylene-2-tricyclo[6.2.1.0^{1,5}]undecanyl]methyl ester | 52771-09-2 |
| VIGOFLOR | spiro[oxolane-2,8'-tricyclo[5.2.1.0^{2,6}]decane] | 68480-11-5 |
| YLANGANATE | benzoic acid, 2-methyl-, methyl ester | 89-71-4 |
| YARA YARA | naphthalene, 2-methoxy- | 93-04-9 |
| Zestoril | 2-propanethiol, 2-(4-methyl-1-cyclohexenyl)- | 61758-19-8 |
| ANGELICA ROOT OIL | Natural oil, no chemical name | |
| ARMOISE OIL PURE | Natural oil, no chemical name | |
| ANISE SEED OIL SPANISH | Natural oil, no chemical name | |
| ANGELICA SEED OIL | Natural oil, no chemical name | |
| ARMOISE ESS ROBT | Natural oil, no chemical name | |
| ASAFETIDA ROOT OIL | Natural oil, no chemical name | |
| BASIL OIL GRAND VERT LMR | Natural oil, no chemical name | |
| BASIL OIL VERBENA LMR | Natural oil, no chemical name | |
| BASIL OIL VIETNAM LMR | Natural oil, no chemical name | |
| BLKCURNT BUD ABS LMR FLG FOR LIFE | Natural oil, no chemical name | |
| BASIL OIL SWEET | Natural oil, no chemical name | |
| BASIL ABS GRAND VERT LMR | Natural oil, no chemical name | |
| BASIL ABS GRAND VERT MD LMR | Natural oil, no chemical name | |
| BIRCH TAR RECT EXTRA | Natural oil, no chemical name | |
| BASIL OIL | Natural oil, no chemical name | |
| BUCHU LEAF OIL BETULINA | Natural oil, no chemical name | |
| Blackcurrant Bud Abs | Natural oil, no chemical name | |
| BUCHU LEAF OIL BETULINA | Natural oil, no chemical name | |
| CADE OIL RECT | Natural oil, no chemical name | |
| CHAMOMILE OIL ROMAN LMR SFO | Natural oil, no chemical name | |
| CINNAMON BARK OIL CEYLON LMR | Natural oil, no chemical name | |
| COFFEE OIL FILT BRAZ. | Natural oil, no chemical name | |
| CARDAMOM OIL GUATEMALA | Natural oil, no chemical name | |
| CYPRIOL | Natural oil, no chemical name | |
| CARDAMOM GUAT EXTRACT CO2 LMR | Natural oil, no chemical name | |
| CARDAMOM OIL GUATEMALA LMR | Natural oil, no chemical name | |
| CARDAMOM OIL EI LMR | Natural oil, no chemical name | |
| CHAMOMILE OIL ENG | Natural oil, no chemical name | |
| CASTOREUM RESOID LMR | Natural oil, no chemical name | |
| CISTE ABS LMR | Natural oil, no chemical name | |
| CHAMOMILE OIL WILD LMR | Natural oil, no chemical name | |
| CISTE ABS C'LESS LMR | Natural oil, no chemical name | |
| COFFEE ABS ARABICA CO2 | Natural oil, no chemical name | |
| CHAMOMILE OIL WILD LOW LIM REF A LMR | Natural oil, no chemical name | |
| CYPRIOL OIL LMR | Natural oil, no chemical name | |
| CYPRIOL HEART LMR | Natural oil, no chemical name | |
| CINNAMON BARK ESSENTIAL LMR | Natural oil, no chemical name | |
| CUMIN SEED OIL PURE | Natural oil, no chemical name | |
| DAVANA OIL LMR FLG SFO | Natural oil, no chemical name | |
| DYNAMONE SB | Natural oil, no chemical name | |
| EUCALYPTUS OIL 80/85 NP | Natural oil, no chemical name | |
| TARRAGON OIL PURE | Natural oil, no chemical name | |
| EUCALYPTUS OIL 80/85 TOCO | Natural oil, no chemical name | |
| FIR BALSAM ABS | Natural oil, no chemical name | |
| FIR BALSAM ABS RESIN | Natural oil, no chemical name | |
| Garlic Oil NAT EGYPT 1% DEP | Natural oil, no chemical name | |
| GALBANOL LMR | Natural oil, no chemical name | |
| GALBANUM OIL LMR | Natural oil, no chemical name | |
| GALBANUM RESOID LMR | Natural oil, no chemical name | |
| GALBANUM HEART LMR | Natural oil, no chemical name | |
| HELICHRYSUM OIL | Natural oil, no chemical name | |
| HYDRO CARBON RESIN SB | Natural oil, no chemical name | |
| IRONES EX ORRIS TECH LMR | Natural oil, no chemical name | |
| IMMORTELLE ABS BALKANS LMR | Natural oil, no chemical name | |
| JASMIN ABS INDIA MD LMR | Natural oil, no chemical name | |
| JASMIN ABS MAROC LMR | Natural oil, no chemical name | |
| JASMIN ABS SAMBAC MD LMR | Natural oil, no chemical name | |
| JASMIN ABS INDIA LMR | Natural oil, no chemical name | |
| JONQUILLE ABS FRANCE LMR | Natural oil, no chemical name | |
| JASMIN ABS SAMBAC LMR | Natural oil, no chemical name | |
| JASMIN ABS EGYPT LMR | Natural oil, no chemical name | |
| CISTE ABS SIS F0785 | Natural oil, no chemical name | |
| Leatherwood LMR XBOX | Natural oil, no chemical name | |
| LOVAGE OIL | Natural oil, no chemical name | |
| NARCISSE ABS FRENCH LMR | Natural oil, no chemical name | |
| Natrotar Rect BLO | Natural oil, no chemical name | |
| NARCISSE ABS FRENCH CO2 LMR | Natural oil, no chemical name | |
| ORANGE FLOWER ABS MOROCCO LMR | Natural oil, no chemical name | |
| ORANGE FLOWER WATER ABS TUNISIA LMR | Natural oil, no chemical name | |
| ORANGE FLOWER ABS TUNISIA LMR | Natural oil, no chemical name | |
| OSMANTHUS ABS LMR | Natural oil, no chemical name | |
| ORIGANUM OIL TURKISH | Natural oil, no chemical name | |
| ORRIS RESOID LMR | Natural oil, no chemical name | |
| ORRIS CRET MOROCCO FOR LMR | Natural oil, no chemical name | |
| ORRIS MOROC NAT 15PCT 4117C LMR | Natural oil, no chemical name | |
| ORRIS CRET 8 PCT IRONE LMR | Natural oil, no chemical name | |
| ORRIS ABS PALLIDA LMR | Natural oil, no chemical name | |
| Onion Oil NAT 1% ETOH | Natural oil, no chemical name | |
| ORANGE FLOWER ABS BLO | Natural oil, no chemical name | |
| PEPPER PINK CO2 LMR | Natural oil, no chemical name | |
| PATCHOULI OIL LIGHT BLO | Natural oil, no chemical name | |
| PEPPERMINT NAT SLCT | Natural oil, no chemical name | |
| PEPPER OIL BLACK SFO | Natural oil, no chemical name | |
| MINT OIL CRUDE ARVENSIS | Natural oil, no chemical name | |
| ROSE OIL TURKISH LOW METH EUGENOL LMR | Natural oil, no chemical name | |
| ROSE ABS TURK LOW METH EUG LMR | Natural oil, no chemical name | |
| ROSE OIL ISPARTA LOW ME LMR FOR LIFE | Natural oil, no chemical name | |
| ROSE CENTIFOLIA ABS MAROC LMR | Natural oil, no chemical name | |
| ROSE ESSENTIAL LMR XBOX FOR LIFE | Natural oil, no chemical name | |
| ROSE ESSENTIAL LOW ME LMR FOR LIFE | Natural oil, no chemical name | |
| ROSE ABS MD LMR | Natural oil, no chemical name | |
| ROSE ABS MAROC LMR | Natural oil, no chemical name | |
| ROSE OIL TURKISH LMR | Natural oil, no chemical name | |
| ROSE OIL BULGARIAN LMR | Natural oil, no chemical name | |
| ROSE ABS TURKISH LMR | Natural oil, no chemical name | |
| ROSE ABS VAH DIJON BLO | Natural oil, no chemical name | |
| ROSE ABS DAMASCENA PURE BLO | Natural oil, no chemical name | |
| SINENSAL NATURAL 20 EX ORANGE | Natural oil, no chemical name | |
| SCHINUS MOLLE OIL LMR SFO | Natural oil, no chemical name | |
| SCHINUS MOLLE ABS MD LMR | Natural oil, no chemical name | |
| SCHINUS MOLLE CO2 EXTRACT | Natural oil, no chemical name | |
| SPEARMINT OIL PURE | Natural oil, no chemical name | |
| STYRAX ESS PYROGENEE SB | Natural oil, no chemical name | |
| TOBAC ABS BALKAN LOW NICOTINE | Natural oil, no chemical name | |
| TAGETTE OIL MD LMR | Natural oil, no chemical name | |
| TAGETTE OIL EGYPT LMR | Natural oil, no chemical name | |
| THYME OIL WHITE SPAIN BLO | Natural oil, no chemical name | |
| TAGETE OIL MADAGASCAR LMR | Natural oil, no chemical name | |
| TUBEROSE ABS INDIA LMR | Natural oil, no chemical name | |
| VIOLET LEAF ABS EGYPT LMR | Natural oil, no chemical name | |
| VANILLA BEAN ABS MADAG | Natural oil, no chemical name | |
| WINTERGREEN OIL . | Natural oil, no chemical name | |

2. The consumer product of claim 1, wherein the High-Performance fragrance composition comprises at least 60% by weight of two or more of High-Performance fragrance ingredients selected from the group consisting of the High-Performance fragrance ingredients listed in Table 2.

3. The consumer product of claim 1, wherein the High-Performance fragrance composition comprises at least 75% by weight of five or more of High-Performance fragrance ingredients selected from the group consisting of the High-Performance fragrance ingredients listed in Table 2.

4. The consumer product of claim 1, wherein the High-Performance fragrance composition comprises at least 90% by weight of seven or more of High-Performance fragrance ingredients selected from the group consisting of the High-Performance fragrance ingredients listed in Table 2.

5. The consumer product of claim 1, wherein said product is a personal care product, fabric care product, or home fragrance product.

6. The consumer product of claim 1, wherein the consumer product is a body wash which exhibits a clarity of less than 20 Nephelometric Turbidity Units.

7. The consumer product of claim 6, wherein clarity is maintained for at least a month after storage at 45°C.

8. The consumer product of claim 1, wherein the consumer product is a body wash which exhibits a viscosity in the range of 10000 and 12000 mPas.

9. The consumer product of claim 8, wherein viscosity is maintained for at least a month after storage at 45°C.

10. The consumer product of claim 1, wherein the consumer product is an antiperspirant, a deodorant, a scent booster, or a liquid detergent which exhibits reduced discoloration.

11. The consumer product of claim 1, wherein the consumer product is a powder detergent which exhibits reduced caking.

12. The consumer product of claim 1, wherein the consumer product is a fabric conditioner and the consumer product active is at a level between 1% and 20% by weight of the consumer product.

13. The consumer product of claim 1, wherein the consumer product is a candle which exhibits reduced soot and volatile organic compound production.

14. The consumer product of claim 10, wherein the consumer product is an antiperspirant or a deodorant which masks a malodor.
